# EUROPÄISCHE PATENTSCHRIFT

(11) **EP 0 611 767 B1**
(45) Veröffentlichungstag und Bekanntmachung des Hinweises auf die Patenterteilung: **06.09.2000**
(21) Anmeldenummer: 94101543.0
(22) Anmeldetag: 02.02.1994
(51) Int. Cl.: C07D 471/04, A61K 31/435, A61K 31/415, C07D 235/08, C07D 235/18, A61K 31/40, A61K 31/41

(54) **Heterocyclisch substituierte Phenyl-cyclohexan-carbonsäurederivate als Angiotensin II Antagonisten**
Heterocyclic substituted phenyl-cyclohexan carboxylic acid derivatives as angiotensin II antagonists
Dérivés d'acides carboxyliques de cyclohexane phényle substitués par hétérocycles

(30) Priorität: 15.02.1993 DE 4304455
(43) Veröffentlichungstag der Anmeldung: 24.08.1994
(73) Patentinhaber: BAYER AG, 51368 Leverkusen (DE)
(72) Erfinder: Müller, Ulrich E., Dr., D-42111 Wuppertal (DE); Dressel, Jürgen, Dr., D-42477 Radevormwald (DE); Fey, Peter, Dr., D-42111 Wuppertal (DE); Hanko, Rudolf H., Dr., D-40237 Düsseldorf (DE); Hübsch, Walter, Dr., D-42113 Wuppertal (DE); Krämer, Thomas, Dr., D-42111 Wuppertal (DE); Müller-Gliemann, Matthias, Dr., D-42697 Solingen (DE); Beuck, Martin, dr., D-40699 Erkrath (DE); Kazda, Stanislav, Prof. Dr., D-42115 Wuppertal (DE); Wohlfeil, Stefan, Dr., D-40724 Hilden (DE); Knorr, Andreas, Dr., D-40699 Erkrath (DE); Stasch, Johannes-Peter, Dr., D-42651 Solingen (DE); Zaiss, Siegfried, Dr., D-42113 Wuppertal (DE)

(56) Entgegenhaltungen:
- EP-A- 0 470 543
- EP-A- 0 513 533
- DE-A- 4 031 635

## Beschreibung

Die Erfindung betrifft heterocyclisch-substituierte Phenyl-cyclohexan-carbonsäurederivate, Verfahren zu ihrer Herstellung sowie ihre Verwendung in Arzneimitteln, insbesondere als blutdrucksenkende und antiatheroslderotische Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigerndes Oktapeptid Angiotensin 11 abgebaut wird. Die verschiedenen Effekte des Angiotensin 11, wie beispielsweise Vasokonstriktion, Na⁺⁻Retention in der Niere, Aldosteronfreisetzung in der Nebenniere und Tonuserhöhung des sympathischen Nervensystems wirken synergistisch im Sinne einer Blutdruckerhöhung.

Darüberhinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Außerdem sind aus den Publikationen EP 407 102, EP 399 731, EP 399 732, EP 324 377 und EP 253 310 heterocyclische Verbindungen mit A II-antagonistischer Wirkung bekannt.

Die DE-A-4 031 635 offenbart Verbindungen mit Angiotensin-antagonistischer Wirkung, die sich strukturell von den Verbindungen der vorliegenden Erfindung durch das Fehlen eines Spacers und das Fehlen von Substituenten an Benzimidazolrest der Verbindungen unterscheiden.

Die EP-A-0 470 543 betrifft Imidazole, die als Angiotensin-Antagonisten wirken, sich aber durch die Substitution in 4'-Position des 1-Benzyl-benz/pyridoimidazols wesentlich von den erfindungsgemäßen Verbindungen unterscheiden.

Die Erfindung betrifft jetzt heterocyclisch substituierte Phenyl-cyclohexan-carbonsäurederivate der allgemeinen Formel (I) 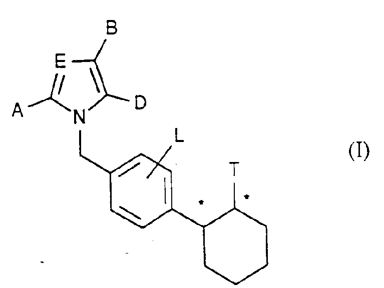 in welcher
A für Wasserstoff oder für Aryl mit 6 bis 10 Kohlenstoffatomen steht, das gegebenenfalls durch Hydroxy, Halogen, Trifluormethyl oder durch geradkettiges oder verzweigtes Alkyl oder Alkoxy mit jeweils bis zu 6 Kohlenstoffatomen substiutiert ist, oder
   für geradkettiges oder verzweigtes Alkyl oder Alkenyl mit jeweils bis zu 8 Kohlenstoffatomen steht, oder
   für Cycloalkyl mit 3 bis 8 Kohlenstoffatomen steht,
E für ein Stickstoffatom oder für einen Rest der Formel -CR¹ steht, worin
R¹ die oben angegebene Bedeutung von A hat und mit dieser gleich oder verschieden ist,
B und D gemeinsam einen Rest der Formel 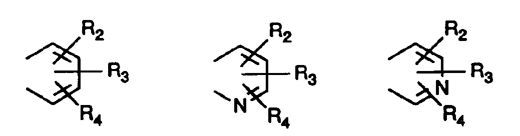 bilden, 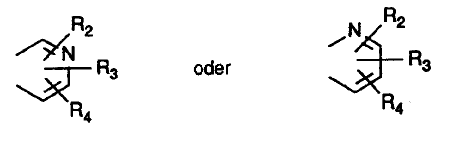 worin
R², R³ und R⁴ gleich oder verschieden sind und die oben angegebene Bedeutung von A haben und mit dieser gleich oder verschieden sind, oder Halogen bedeuten,
   L für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,
T für einen Rest der Formel 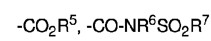 oder 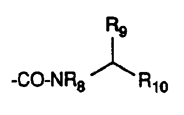 steht,
   worin
R⁵ Wasserstoff, geradkettiges oder verzweigtes Alkyl mit bis zu 8 Kohlenstoffatomen, Cycloalkyl mit 3 bis 7 Kohlenstoffatomen oder Phenyl bedeutet,
R⁶ und R⁸ gleich oder verschieden sind und Wasserstoff oder geradkettiges oder verzweigtes Alkyl mit bis zu 6 Kohlenstoffatomen bedeuten,
R⁷ Trifluormethyl oder geradkettiges oder verzweigtes Alkyl mit bis zu 6 Kohlenstoffatomen, Benzyl oder Phenyl bedeutet, die gegebenenfalls durch geradkettiges Alkyl mit bis zu 6 Kohlenstoffatomen substituiert sind,
R⁹ Aryl mit 6 bis 10 Kohlenstoffatomen bedeutet, das gegebenenfalls bis zu 2-fach gleich oder verschieden durch Halogen, Hydroxy, geradkettiges oder verzweigtes Alkyl, Alkoxy oder Alkoxycarbonyl mit jeweils bis zu 6 Kohlenstoffatomen, Carboxy, Phenoxy oder C₃-C₆-Cycloalkyloxy substituiert ist,
R¹⁰ eine Gruppe der Formel -CH₂-OR¹¹, -CO2R12, -CO-NR¹³R¹⁴ oder Pyridyl bedeutet, worin
R¹¹ Wasserstoff oder geradkettiges oder verzweigtes Alkyl mit bis zu 8 Kohlenstoffatomen bedeutet,
R¹² Wasserstoff, geradkettiges oder verzweigtes Alkyl mit bis zu 8 Kohlenstoffatomen, Phenyl oder Cycloalkyl mit 3 bis 6 Kohlenstoffatomen bedeutet,
R¹³ und R¹⁴ gleich oder verschieden sind und Wasserstoff, geradkettiges oder verzweigtes Alkyl mit bis zu 8 Kohlenstoffatomen oder Phenyl bedeuten,
   oder
T für Tetrazolyl steht, das gegebenenfalls durch Triphenylmethyl oder geradkettiges oder verzweigtes Alkyl mit bis zu 4 Kohlenstoffatomen substituiert ist,

gegebenenfalls in einer stereoisomeren Form, und deren physiologisch unbedenkliche Salze.

Die erfindungsgemäßen Verbindungen der allgemeinen Formel (I) können auch in Form ihrer physiologisch unbedenklichen Salze vorliegen. Im allgemeinen seien hier Salze mit organischen oder anorganischen Basen oder Säuren genannt.

Physiologisch unbedenkliche Salze der neuen heterocyclisch substituierten Phenyl-cyclohexancarbonsäuren und -carbonsäurederivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher
A für Wasserstoff oder für Phenyl steht, das gegebenenfalls durch Hydroxy, Fluor, Chlor, Brom, Trifluormethyl oder durch geradkettiges oder verzweigtes Alkyl oder Alkoxy mit jeweils bis zu 4 Kohlenstoffatomen substituiert ist, oder
   für geradkettiges oder verzweigtes Alkyl oder Alkenyl mit jeweils bis zu 6 Kohlenstoffatomen steht, oder
   für Cyclopropyl, Cyclobutyl, Cyclopentyl, Cyclohexyl oder Cycloheptyl steht,
E für ein Stickstoffatom oder für einen Rest der Formel -CR¹ steht,
   worin
R¹ die oben angegebene Bedeutung von A hat und mit dieser gleich oder verschieden ist,
B und D gemeinsam einen Rest der Formel 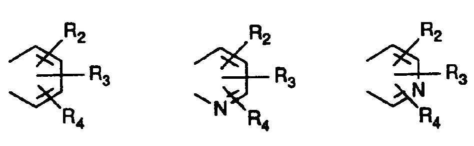 bilden, 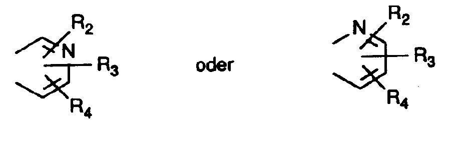 worin
R², R³ und R⁴ gleich oder verschieden sind und die oben angegebene Bedeutung von A haben und mit dieser gleich oder verschieden sind, oder Fluor, Chlor oder Brom bedeuten,
L für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,
T für einen Rest der Formel 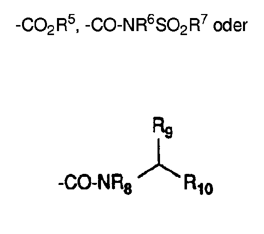 steht,
   worin
R⁵ Wasserstoff, geradkettiges oder verzweigtes Alkyl mit bis zu 6 Kohlenstoffatomen, Cyclopropyl, Cyclopentyl, Cyclohexyl oder Phenyl bedeutet,
R⁶ und R⁸ gleich oder verschieden sind und Wasserstoff oder geradkettiges oder verzweigtes Alkyl mit bis zu 4 Kohlenstoffatomen bedeuten,
R⁷ Trifluormethyl oder geradkettiges oder verzweigtes Alkyl mit bis zu 4 Kohlenstoffatomen, Benzyl oder Phenyl bedeutet, die gegebenenfalls durch geradkettiges oder verzweigtes Alkyl mit bis zu 4 Kohlenstoffatomen substituiert sind,
R⁹ Phenyl bedeutet, das gegebenenfalls durch Fluor, Chlor, Brom, Hydroxy, geradkettiges oder verzweigtes Alkyl, Alkoxy oder Alkoxycarbonyl mit jeweils bis zu 4 Kohlenstoffatomen, Carboxy oder Phenoxy substituiert ist,
R¹⁰ eine Gruppe der Formel -CH₂-OR¹¹, -C0₂R¹², -CO-NR¹³R¹⁴ oder Pyridyl bedeutet, worin
R¹¹ Wasserstoff oder geradkettiges oder verzweigtes Alkyl mit bis zu 6 Kohlenstoffatomen bedeutet,
R¹² Wasserstoff, geradkettiges oder verzweigtes Alkyl mit bis zu 6 Kohlenstoffatomen, Phenyl, Cyclopropyl, Cyclopentyl oder Cyclohexyl bedeutet,
R¹³ und R¹⁴ gleich oder verschieden sind und Wasserstoff, geradkettiges oder verzweigtes Alkyl mit bis zu 6 Kohlenstoffatomen, Cyclopropyl, Cylcopentyl, Cylcohexyl oder Phenyl bedeuten, oder
T für Tetrazolyl steht, das gegebenenfalls durch Triphenylmethyl oder Methyl substituiert ist,

gegebenenfalls in einer stereoisomeren Form, und deren physiologisch unbedenkliche Salze.

Besonders bevorzugt sind Verbindungen der allgemeinen Formel (I),
in welcher
A für Wasserstoff oder
   für geradkettiges oder verzweigtes Alkyl mit bis zu 4 Kohlenstoffatomen steht, oder
   für Phenyl oder Cyclopropyl, Cyclopentyl oder Cyclohexyl steht,
E für ein Stickstoffatom steht,
B und D gemeinsam einen Rest der Formel 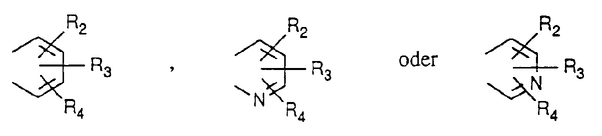 bilden,
   worin
R², R³ und R⁴ gleich oder verschieden sind und die oben angegebene Bedeutung von A haben und mit dieser gleich oder verschieden sind, oder Fluor, Chlor oder Brom bedeuten,
   L für Wasserstoff, Fluor, Chlor, Trifluormethyl oder Methyl steht,
T für einen Rest der Formel 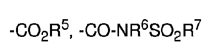 oder 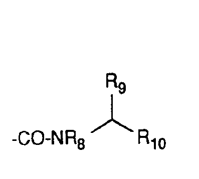 steht,
   worin
R⁵ Wasserstoff, geradkettiges oder verzweigtes Alkyl mit bis zu 4 Kohlenstoffatomen bedeutet,
R⁶ und R⁸ gleich oder verschieden sind und Wasserstoff oder Methyl bedeuten,
R⁷ Trifluormethyl oder Methyl, Ethyl, Benzyl, p-Tolyl oder Phenyl bedeutet,
R⁹ Phenyl bedeutet, das gegebenenfalls durch Fluor, Chlor, Brom, Hydroxy, geradkettiges oder verzweigtes Alkyl, Alkoxy oder Alkoxycarbonyl mit jeweils bis zu 3 Kohlenstoffatomen, Carboxy oder Phenoxy substituiert ist,
R¹⁰ eine Gruppe der Formel -CH₂-OR¹¹, -C0₂-R¹², -CO-NR¹³R¹⁴ oder Pyridyl bedeutet, worin
R¹¹ Wasserstoff oder geradkettiges oder verzweigtes Alkyl mit bis zu 4 Kohlenstoffatomen bedeutet,
R¹² Wasserstoff, geradkettiges oder verzweigtes Alkyl mit bis zu 4 Kohlenstoffaromen bedeutet,
R¹³ und R¹⁴ gleich oder verschieden sind und Wasserstoff, geradkettiges oder verzweigtes Alkyl mit bis zu 4 Kohlenstoffatomen bedeuten.
   oder
T für Tetrazolyl steht, das gegebenenfalls durch Triphenylmethyl oder Methyl substituiert ist,

gegebenenfalls in einer stereoisomeren Form, und deren physiologisch unbedenkliche Salze.

Ganz besonders bevorzugt sind Verbindungen der allgemeinen Formel (I),
in welcher
A für Wasserstoff, geradkettiges oder verzweigtes Alkyl mit bis zu 4 Kohlenstoffatomen oder für Phenyl oder Cyclopropyl steht.
E für ein Stickstoffatom steht,
B und D gemeinsam einen Rest der Formel 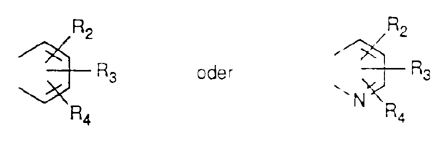 bilden,
   worin
R², R³ und R⁴ gleich oder verschieden sind und Methyl, Wasserstoff, Fluor, Chlor oder Brom bedeuten, L für Wasserstoff, Fluor oder Chlor steht,
T für einen Rest der Formel 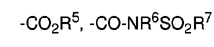 oder 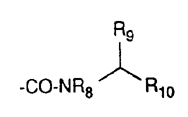 steht,
   worin
R⁵ Wasserstoff oder geradkettiges oder verzweigtes Alkyl mit bis zu 4 Kohlenstoffatomen bedeutet,
R⁶ und R^{B} gleich oder verschieden sind und Wasserstoff oder Methyl bedeuten,
R⁷ Trifluormethyl, Methyl oder p-Tolyl bedeutet,
R⁹ Phenyl bedeutet, das durch Fluor, Chlor substituiert sein kann, und
R¹⁰ eine Gruppe der Formel -CH₂0H, oder -CONH₂ oder Pyridyl bedeutet, oder
T für Tetrazolyl steht, das gegebenenfalls durch Triphenylmethyl substituiert ist,

gegebenenfalls in einer stereoisomeren Form, und deren physiologisch unbedenkliche Salze.

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (II) 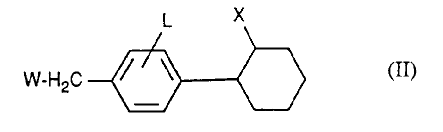 in welcher
L die oben angegebene Bedeutung hat,
W für eine typische Abgangsgruppe wie beispielsweise Chlor, Brom, Jod, Tosylat oder Mesylat, vorzugsweise für Brom steht
   und
X für C₁-C₆-Alkoxycarbonyl oder für die Triphenylmethyl-tetrazolyl-1-yl-Gruppe steht,
   zunächst mit Verbindungen der allgemeinen Formel (III) 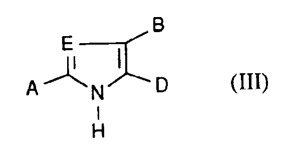 in welcher
A, B, D und E die oben angegebene Bedeutung haben,
   in inerten Lösemitteln, gegebenenfalls in Anwesenheit einer Base und gegebenenfalls unter Schutzgasatmosphäre zu Verbindungen der allgemeinen Formel (IV) 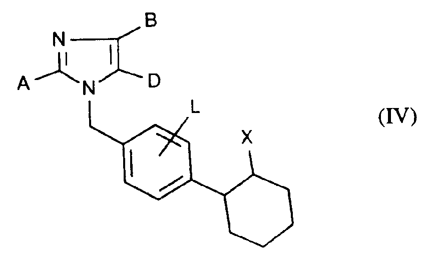 in welcher
A, B, D, L und X die oben angegebene Bedeutung haben,
   umsetzt, und gegebenenfalls ausgehend von den entsprechenden Carbonsäuren, nach vorgeschalteter Hydrolyse und/oder Aktivierung, anschließend mit Sulfonaminen oder Aminen der allgemeinen Formeln (V) und (Va) 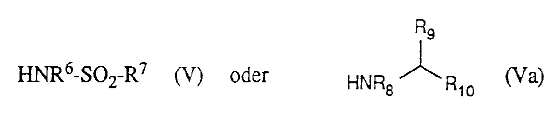 in welchen
R⁶, R⁷, R⁸, R⁹ und R¹⁰ die oben angegebene Bedeutung haben,

gegebenenfalls in Anwesenheit einer Base und/oder eines Dehydratisierungsmittels, in inerten Lösemitteln amidiert, und im Fall der freien Tetrazole, gegebenenfalls die Triphenylmethylgruppe nach üblichen Methoden mit Säuren, vorzugsweise mit Trifluoressigsäure oder Salzsäure in Dioxan, abspaltet,
und gegebenenfalls die Stereoisomeren trennt, und im Fall der Herstellung der physiologisch unbedenklichen Salze mit einer entsprechenden Base oder Säure umsetzt.

Das erfindungsgemäße Verfahren kann durch folgendes Formelschema beispielhaft erläutert werden:

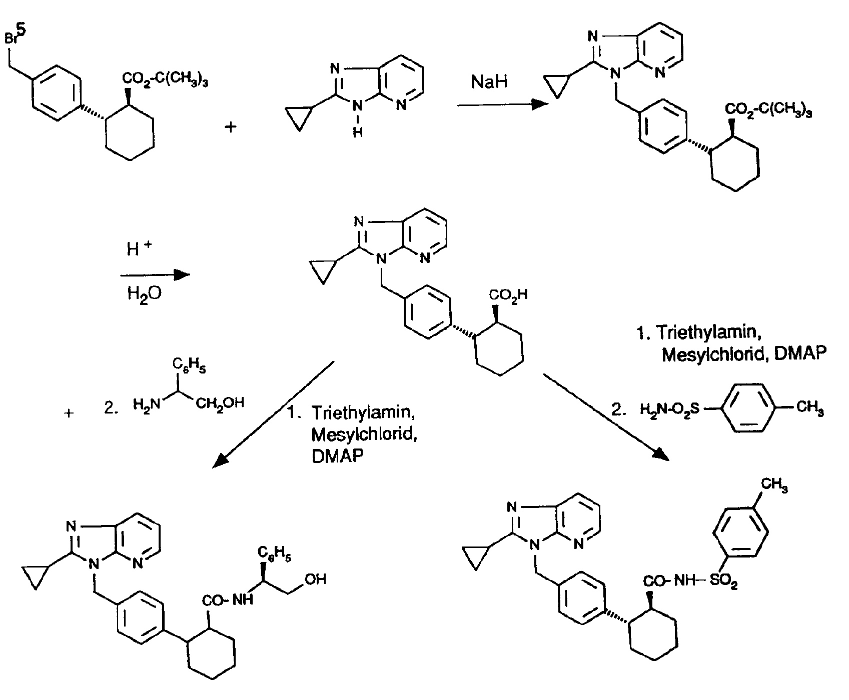

Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid und Tetrahydrofuran.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(Cᵢ-C₆)amine) wie Triethylamin, oder Heterocyclen wie 1,4-Diazabicyclo[2.2.2]octan (DABCO), 1,8-Diazabi- cyclo[5.4.0]undec-7-en (DBU), Pyridin, Diaminopyridin, Methylpiperidin oder Morpholin. Es ist auch möglich als Basen Alkalimetalle wie Natrium oder deren Hydride wie Natriumhydrid einzusetzen. Bevorzugt sind Natriumhydrid, Kaliumcarbonat, Triethylamin, Pyridin und Kalium-tert.butylat, DBU oder DABCO.

Im allgemeinen setzt man die Base in einer Menge von 0,05 mol bis 10 mol, bevorzugt von 1 mol bis 2 mol, bezogen auf 1 mol der Verbindung der Formel (111), ein.

] Das erfindungsgemäße Verfahren wird im allgemeinen in einem Temperaturbereich von -80°C bis +100°C, bevorzugt von -30°C bis +60°C, durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Hydrolyse die üblichen anorganischen Basen. Hierzu gehören bevorzugt Alkalihydroxide oder Erdalkalihydroxide wie beispielsweise Lithiumhydroxid, Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Lithiumhydroxid, Natriumhydroxid oder Kaliumhydroxid eingesetzt.

Als Lösemittel eignen sich für die Hydrolyse Wasser oder die für eine Hydrolyse üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Hydrolyse kann auch mit wäßrigen Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Hydrolyse wird im allgemeinen in einem Temperaturbereich von 0°C bis +100°C, bevorzugt von +20°C bis +80°C durchgeführt.

Im allgemeinen wird die Hydrolyse bei Normaldruck durchgeführt. Es ist aber auch möglich, bei Unterdruck oder bei Überdruck zu arbeiten (z.B. von 0,5 bis 5 bar).

Bei der Durchführung der Hydrolyse wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Säuren wie beispielsweise Chlorwasserstoffsäure, Bromwasserstoffsäure, Schwefelsäure, Phosphorsäure oder Trifluoressigsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden. Im Fall der basischen Heterocyclen können durch das Behandeln der Lösungen der Carboxylate mit den oben aufgeführten Säuren auch die Salze der Heterocyclen mit den anorganischen Säuren gewonnen werden.

Die Amidierung und die Sulfonamidierung der Verbindungen der allgemeinen Formel (IV) erfolgen im allgemeinen in einem der oben aufgeführten Lösemitteln, vorzugsweise in Tetrahydrofuran oder Dichlormethan.

Die Amidierung kann gegebenenfalls über die aktivierte Stufe der Säurehalogenide, die aus den entsprechenden Säuren durch Umsetzung mit Thionylchlorid, Phosphortrichlorid, Phosphorpentachlorid, Phosphortribromid oder Oxalylchlorid hergestellt werden können, verlaufen.

Die Amidierung erfolgt im allgemeinen in einem Temperaturbereich von - 20°C bis +80°C, vorzugsweise von -10°C bis +30°C und Normaldruck.

Als Basen eignen sich dafür neben den oben aufgeführten Basen vorzugsweise Triethylamin und/oder Dimethylaminopyridin, DBU oder DABCO.

Die Base wird in einer Menge von 0,5 mol bis 10 mol, bevorzugt von 1 mol bis 5 mol, bezogen auf 1 mol der Verbindungen der allgemeinen Formeln (IV) und (V) eingesetzt.

Als säurebindende Mittel für die Amidierung können Alkali- oder Erdalkalicarbonate wie Natriumcarbonat, Kaliumcarbonat, Alkali- oder Erdalkalihydroxide wie beispielsweise Natrium- oder Kaliumhydroxid, oder organische Basen wie Pyridin, Triethylamin, N-Methylpiperidin, oder bicyclische Amidine wie 1,5-Diazabicyclo[3.4.0]-nonene-5 (DBN) oder 1,5-Diazabicyclo[3.4.0]undecene-5 (DBU) eingesetzt werden. Bevorzugt ist Kaliumcarbonat.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium- hexylfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren eingesetzt.

Die Cyclohexan-Verbindungen der allgemeinen Formel (11) sind größtenteils neu und können hergestellt werden, indem man Verbindungen der allgemeinen Formel (VI) 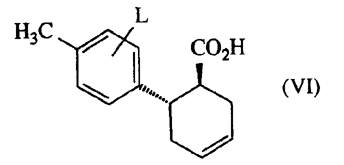 in welcher
L die oben angegebene Bedeutung hat,
   zunächst durch Hydrierung mit Palladium/C in einem der oben angegebenen Lösemitteln, vorzugsweise Methanol, in einer Wasserstoffatmosphäre in die Verbindungen der allgemeinen Formel (VII) 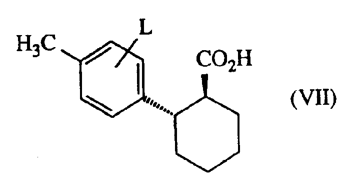 in welcher
L die oben angegebene Bedeutung hat,
   überführt,
   in einem zweiten Schritt, im Fall, daß T Tetrazot, nach üblichen Methoden verestert,
   und im Fall, daß T für den Tetrazolylrest steht, mit Chlorsulfonylisocyanat in Dichlormethan zu den entsprechenden Cyanoverbindungen umsetzt, anschließend mit Natriumazid / Triethylammoniumchlorid, in Anwesenheit einer der oben aufgeführten Basen, vorzugsweise N,N-Dimethylformamid, unter Stickstoffatmosphäre die Tetrazolylgruppe einführt, durch weitere Umsetzung mit Triphenylmethylchlorid in Anwesenheit eines der oben aufgeführten Lösemittel und Basen, vorzugsweise Dichlormethan und Triethylamin, die Triphenylmethylgruppe einführt,
   und in einem letzten Schritt an der Methylengruppe eine Bromierung, gegebenenfalls in Anwesenheit eines Katalysators, durchführt.

Die Reduktion der Doppelbindung erfolgt in einem Temperaturbereich von 0°C bis +40°C, vorzugsweise bei +20°C und einem Druck von 1 bar, -10 bar, vorzugsweise 1-3 bar.

Die Veresterung erfolgt in einem der oben angegebenen Lösemitteln, vorzugsweise Toluol und Tetrahydrofuran, nach der oben bereits beschriebenen vorgeschalteten Aktivierung der entsprechenden Carbonäsure, vorzugsweise über die Carbonsäurechloride, und nachträglicher Umsetzung mit dem entsprechenden Alkoholaten, in einem Temperaturbereich von 0°C bis +60°C, vorzugsweise bei +10°C bis +35°C und Normaldruck.

Die Umsetzungen zu den Cyanoverbindungen und den Tetrazolylverbindungen werden im allgemeinen bei der Siedetemperatur des jeweiligen Lösemittels und Normaldruck durchgeführt.

Die Einführung der Triphenylmethylgruppe in den Tetrazolylring erfolgt im allgemeinen bei 0°C.

Die Bromierung wird im allgemeinen in einem Temperaturbereich von +40°C bis 100°C, vorzugsweise von +60°C bis +90°C und Normaldruck durchgeführt. Sie erfolgt in einem der oben angegebenen Lösemitteln, vorzugsweise mit Tetrachlorkohlenstoff, und mit N-Bromsuccinimid.

Als Starter (Katalysator) für die Bromierung eignen sich beispielsweise Azobisisobutyronitril, Dibenzoylperoxid, vorzugsweise Azobisisobutyronitril, wobei der Starter in einer Menge von 0,01 mol bis 0,1 mol, bevorzugt von 0,01 mol bis 0,05 mol, bezogen auf 1 mol der Verbindung der allgemeinen Formel (VII), eingesetzt wird.

Die Verbindungen der allgemeinen Formel (VI) sind ebenfalls neu und können beispielsweise hergestellt werden, indem man Verbindungen der allgemeinen Formel (VIII) 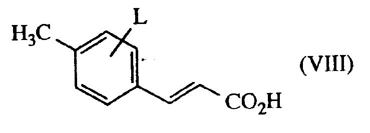 in welcher
L die oben angegebene Bedeutung hat,

in einem der oben angegebenen Lösemittel, vorzugsweise Toluol, mit 1,3-Butadien in Anwesenheit von Hydrochinon, in einem Temperaturbereich von +180°C bis +230°C, vorzugsweise bei 200°C und einem Druck von ca. 20 bar umsetzt [vgl. hierzu Eur.J. Med. Chem. 11, 493 (1976)].

Die Verbindungen der allgemeinen Formel (VII sind an sich bekannt oder können nach üblichen Methoden hergestellt werden.

Die Verbindungen der allgemeinen Formeln (IV) und (VII) sind neu und können beispielsweise nach den oben beschriebenen Verfahren hergestellt werden.

Ebenso sind die Verbindungen der allgemeinen Formel (III) an sich bekannt oder können nach üblicher Methode hergestellt werden.

Die Amine der allgemeinen Formel (V) sind bekannt oder können nach bekannten Verfahren hergestellt werden.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie kompetitiv die Bindung von Angiotensin II an die Rezeptoren hemmen. Sie unterdrücken die vasokonstriktorischen und Aldosteronsekretions-stimulierenden Effekte des Angiotensin 11. Darüberhinaus inhibieren sie die Proliferation von glatten Muskelzellen.

Sie können deshalb in Arzneimitteln zur Behandlung der arteriellen Hypertonie und Atherosklerose eingesetzt werden. Darüberhinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

### Untersuchung auf die Hemmung der mit Agonisten induzierten Kontraktion

Kaninchen beiderlei Geschlechts werden durch Nackenschlag betäubt und entblutet, oder fallweise mit Nembutal (ca 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Orgapbäder mit auf 37°C temperierter, Carbogen-begaster Krebs-Henseleit-Nährlösung folgender Zusammensetzung: 119 mmol/l NaCI; 2,5 mmol/I CaC1₂ x 2 H₂0; 1,2 mmol/l KH₂P0₄; 10 mmol/l Glucose; 4,8 mmol/l KCI; 1,4 mmol/I MgS0₄ x 7 H₂0 und 25 mmol/l NaHC0₃ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzetkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

### Agonisten und ihre Standardkonzentrationen Applikationsvolumen pro Einzelgabe = 100 µl)

[0056]

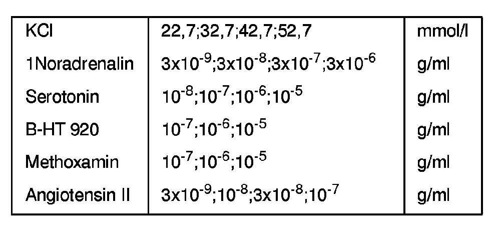

Für die Berechnung der IC₅₀ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

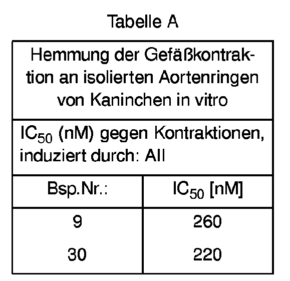

### Blutdruckmessuncen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

### Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenanerienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht.
Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

### Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membrankfraktionen partiell aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), ³H-Angiotensin II (3-5 nM), Test-Pufferlösung (50 mM Tris, pH 7,2, 5 mM MgCl₂ sowie die zu untersuchenden Substanzen enthält Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCI, pH 7,4, 5% PEG 6000) in einem Szintillationscocktail spektrophotometrisch gemessen. Die Analyse der Rohdaten erfolgte mit Computer-Programmen zu K- bzw. IC_{5O}-Werten (Kᵢ: für die verwendete Radioaktivität korrigierte IC₅₀-Werte; IC₅₀-Werte: Konzentration bei der die zu untersuchende Substanz eine 50%ige Hemmung der spezifischen Bindung des Radioliganden bewirkt).

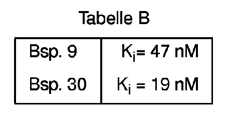

### Untersuchung zur Inhibition der Proliferation glatter Muskelzellen durch die erfindungsgemäßen Verbindungen

Zur Feststellung der antiproliferativen Wirkung der Verbindungen werden glatte Muskelzellen verwendet, die aus den Aorten von Ratten durch die Media-Explantat-Technik gewonnen werden [R. Ross, J. Cell. Biol. 50, 172, 1971]. Die Zellen werden in geeigneten Kulturschalen, in der Regel 96-Loch-Platten, ausgesät und für 2 - 3 Tage in Medium 199 mit 7,5% FCS und 7,5% NCS, 2 mM L-Glutamin und 15 mM HEPES, pH 7,4 in 5% C0₂ bei 37°C kultiviert. Danach werden die Zellen durch Serumentzug für 2 - 3 Tage synchronisiert und sodann mit Serum oder anderen Faktoren zum Wachstum angeregt. Gleichzeitig werden Testverbindungen zugesetzt. Nach 16 - 20 Stunden wird 1 µCi ³H-Thymidin zugefügt und nach weiteren 4 Stunden der Einbau dieser Substanz in die TCA-präzipitierbare DNA der Zellen bestimmt. Zur Bestimmung der IC₅₀-Werte wird die Wirkstoffkonzentration errechnet, die bei sequentieller Verdünnung des Wirkstoffes halbmaximale Hemmung der durch 10% FCS hervorgerufene Thymidinkorporation bewirkt.

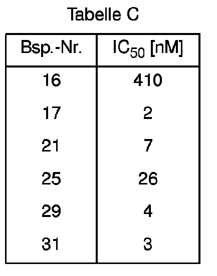

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### Laufmittel:

A Dichlormethan : Methanol = 10:1
B Petrolether : Essigester = 4:1
C Petrolether : Essigester = 1:1
D Dichlormethan : Methanol = 10:1
E Essigester : Dichlormethan = 1:1
F Dichlormethan : Ethanol = 20:1
G Petrolether : Essigester = 1:2
H Dichlormethan : Methanol: wäßr. konz. Ammoniak = 200:20:1

### Ausgangsverbindungen

### Beispiel

### trans-6-(4-Tolyl)-cyclohex-3-en-1-carbonsäure

[0071]

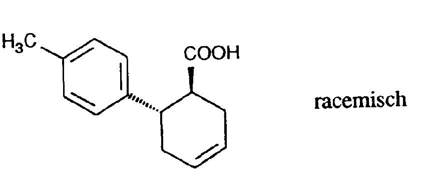

275 g (1,695mo1) 3-E-(4-Tolyl)acrylsäure (käuflich) wird nach einem bekannten Verfahren (Eur.J. Med. Chem. 11, 493 (1976)) in 480 ml Toluol mit 580 ml 1,3-Butadien (in kondensierter Form abgemessen) unter Zugabe von 3 g Hydrochinon 22 h bei ca 200°C und ca. 20 bar umgesetzt. Das Rohgemisch wird mit Toluol verdünnt und mit 0,5 M wäßriger Natronlauge extrahiert. Darauf werden die wäßrigen Phasen mit 1 M Salzsäure angesäuert und mit Ether extrahiert. Die etherischen Lösungen werden mit Natriumsulfat getrocknet, eingedampft und wiederum in Toluol gelöst. Nach 15 minütigem Kochen mit 5g Aktivkohle saugt man heiß ab und dampft das Lösemittel bis auf ca. 120 - 160 ml ab; bei 0 - 4°C kristallisieren 124 g (573 mmol) Produkt aus. Das Filtrat wird etwas weiter eingeengt und zum Nachkristallisieren wieder abgekühlt. Bei Wiederholung dieses Vorgangs fallen insgesamt weitere 42 g (194 mmol) Produkt an. R_{f} = 0,39 (Dichlormethan : Methanol = 10:1)

### Beispiel II

### trans-2-(4-Tolyl)-cyclohexan-1-carbonsäure

[0073]

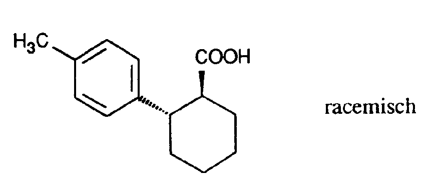

155 g (717 mmol) der Verbindung aus Beispiel I werden in 1 I Methanol gelöst und an 10 g Palladium (10% auf Tierkohle) bei 20°C und Wassestoffatmosphäre von ca 1 bar umgestzt. Nach einer Reaktionszeit von insgesamt 16 h wird der Katalysator abfiltriert und das Lösemittel - zuletzt im Vakuum - abgedampft.
Ausbeute: 153 g (701 mmol)
R_{f} = 0,38 (Dichlormethan : Methanol = 10:1)

### Beispiel III

### trans-2-(4-Tolyl)-cyclohexan-1-carbonsäure-tert.butyleste

[0075]

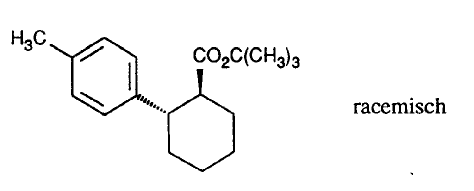

### Methode A:

45,8 g (184 mmol) der Verbindung aus Beispiel II werden in 600 ml Toluol gelöst und unter Rückfluß mit 49,5 ml (387 mmol) Oxalylchlorid zur Reaktion gebracht.

Nach 2 h dampft man das Lösemittel mit überschüssigem Reagenz ab, dazu muß das rohe Carbonsäurechlorid gegebenenfalls wiederholt in Toluol aufgenommen und noch einmal abrotiert werden. Das so gewonnene Produkt wird in 500 ml Tetrahydrofuran gelöst, mit 24,8 g (221 mmol) Kalium-tert.butanolat bei 0°C verrührt und 20 h nachgerührt (bei 20°C). Darauf werden Wasser und Ether zugesetzt und mehrfach extrahiert. Die organische Phase wird mit Natriumsulfat getrocknet, eingedampft und der Rückstand an Kieselgel 60 (Merck, Petrolether : Essigester = 20.1) chromatographisch gereinigt.
Ausbeute: 39,6 g (130 mmol)
R_{f} = 0,47 (Petrolether : Essigester = 10:1)

### Methode B

20,0 g (91,6 mmol) der Verbindung aus Beispiel II werden in 7 ml konz. Schwefelsäure in 100 ml Ether suspendiert und bei -30°C mit 80 ml (713 mmol) Isobuten versetzt (Druckapparatur). Das Gemisch wird in dem verschlossenen Gefäß auf 20°C erwärmt und über 20 Stunden umgesetzt. Darauf kühlt man wieder auf -30°C ab, öffnet die Apperatur und rührt das Reaktionsgemisch bei 20°C in 300 ml 3 M Natronlauge / 400 ml Ether ein. Die wäßrige Phase wird mit Ether nachextrahiert, die organische Lösung mit Natriumsulfat getrocknet und eingedampft. Ausbeute: 23,3 g (84,9 mmol).

### Beispiel IV

### trans-2-(4-Brommethylphenyl)-cyclohexan-1-carbonsäure-tert.butyleste

[0079]

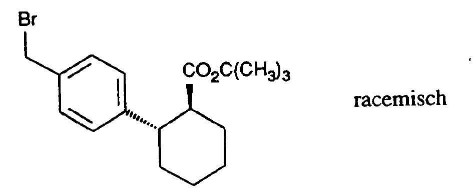

11,70 g (42,6 mmol) der Verbindung aus Beispiel III werden unter Rückfluß in 100 ml Tetrachlormethan mit 7,59 g (42,6 mmol) N-Bromsuccinimid und 1,4 g Azobisisobutyronitril umgesetzt. Nach einer Reaktionszeit von 4 h wird die Mischung abgekühlt, der angefallene Succinimidniederschlag abgesaugt und das Filtrat eingedampft.
Ausbeute: 14,2 g (40,2 mmol)
Rₜ = 0,48 (Petrolether : Essigester = 10:1)

### Beispiel V

### 6-Cyclopropyl-imidazo[2,3-b]pyridin

[0081]

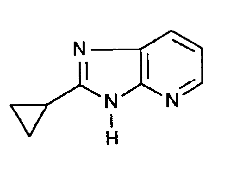

3,27 g (30 mmol) 2,3-Diaminopyridin (Aldrich) und 2,72 g (30 mmol) Cyxclopropancarbonsäure (Aldrich) werden mit 30 ml Tolylphosphorsäure 3 Stunden bei 120°C gerührt. Der Ansatz wird auf Eiswasser gegossen, mit Natriumhydroxid auf pH = 6 - 7 gestellt und mit Natriumcarbonat bis pH = 8-9 basifiziert. Nach mehrfacher Extraktion mit Essigester werden die vereinigten organischen Phasen mit Natriumsulfat getrocknet, filtriert und - zuletzt im Hochvakuum - eingedampft. Nach der chromatographischen Aufarbeitung des Rohproduktes (Kieselgel 60, Merck, Dichlormethan bis Dichlormethan : Methanol = 50:1); Ausbeute: 3,01g (19 mmol).
R_{f}= 0,38 (Essigester : Methanol = 10:1)

### Beispiel VI

### trans-2-(4-Tolyl)-cyclohexan-1-carbonnitril

[0083]

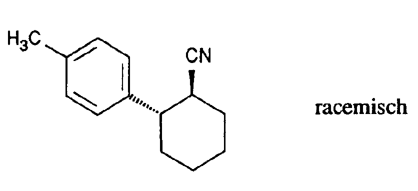

100,0 g (458,0 mmol) der Verbindung aus Beispiel II werden in 1 1 Dichlormethan in der Siedetemperatur mit 84,3 g (595,5 mmol) Chlorsulfonylisocyanat in 100 ml Dichlormethan über 1 h umgesetzt. Darauf tropft man 72 ml (938,9 mmol) N,N-Dimethylformamid in das sich abkühlende Reaktionsgemisch und rührt 18 h nach. Man gießt auf 350 g Eis, trennt die Phasen nach dem Schmelzen und extrahiert mit Dichlormethan. Die mit Kaliumcarbonat getrockneten organischen Phasen werden eingedampft und der Rückstand destilliert. Es fallen 57,8 g (290,2 mmol) Produkt an. Kochpunkt: 122-131°Cc (0,2 mbar)
R_{f} = 0,81 (Dichlormethan)

### Beispiel VII

### 5-[trans-2-(4-Tolyl)-cyclohex-1-yl]tetrazol

[0085]

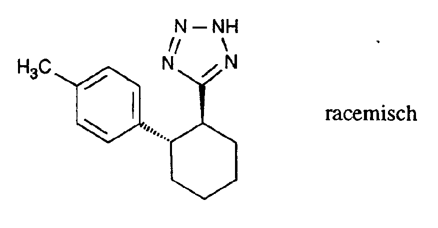

15,34 g (69,6 mmol) der Verbindung aus Beispiel VI werden in 230 ml wasserfreiem N,N-Dimethylformamid mit 22,6 g (348 mol) Natriumazid und 47,9 g (348 mmol) Triethylammmoniumchlorid unter Stickstoff in der Siedehitze zur Reaktion gebracht. Nach 20 h gießt man nach Abkühlung auf Ether und 1 M Schwefelsäure, wäscht die organische Phase mit 1 M Schwefelsäure und extrahiert dann mit 10%iger Natronlauge. Die wäßrige Phase wird bei 0°C mit 1 M Salzsäure auf pH = 1,5 gestellt und der anfallende Niederschlag abgesaugt, mit Wasser gewaschen und im Hochvakuuum über Phosphorpentoxid und Natriumhydroxid getrocknet.
Ausbeute: 11,2 g (46,2 mmol)
R_{f} = 0,23 (Dichlormethan : Methanol = 20:1)

### Beispiel VIII

### 5-[trans-2-(4-Tolyl)-cyclohex-1-yl]-2-triphenylmethyl-tetrazol

[0088]

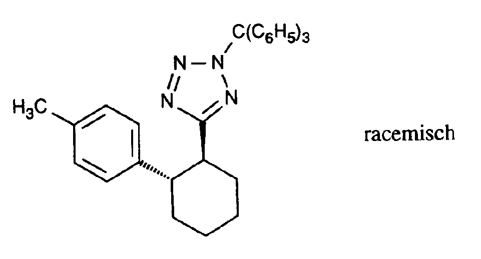

11,0 g (45,7 mmol) der Verbindung aus Beipiel VII weren in 170 ml Dichlormethan mit 13,4 g (48,2 mmol) Triphenylmethylchlorid und 7,57 ml (54,6 mmol) Triehylamin bei 0°C zur Reaktion gebracht. Man rührt unter Erwärmung auf Raumtemperatur ca 20 h nach und extrahiert dann mit Ether und wäßriger Zitronensäure. Die organischen Phasen werden mit Natriumsulfat getrocknet und eingedampft. Ausbeute: 22,1 g (45,5 mmol)
R_{f} = 0,67 (Petrolether : Essigester = 5:1)

### Beispiel IX

### 5-[trans-2-(4-Brommethylphenyl)-cyclohex-1-yl]-2-triphenylmethyl-tetrazol

[0090]

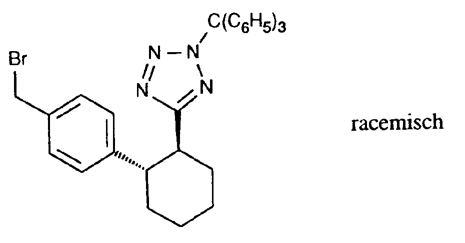

22,1 g (45,5 mmol) der Verbindung aus Beispiel VIII werden in 300 ml Tetrachlormethan mit 8,1 g (45,5 mmol) N-Bromsuccinimid und 0,3 g Azobisisobutyronitril unter Rückfluß umgestezt. Nach 3 stündiger Reaktionszeit kühlt man auf Raumtemperatur und später auf 0°C ab und saugt den Niederschlag ab. Das Filtrat wird eingedampft und man erhält rohes Produkt (26,2 g), das ohne weitere Aufarbeitung weiter umgesetzt wird.
R_{f} = 0,47 (Petrolether : Essigester = 10:1)

### Herstellungsbeispiele

### Beispiel 1

### trans-2-{4-[6-Cyclopropyl-imidazo[2,3-b]pyridin)-7-yl-methyl]-phenyl}-cyclohexan-1-carbonsäure-tert.butylester

[0092]

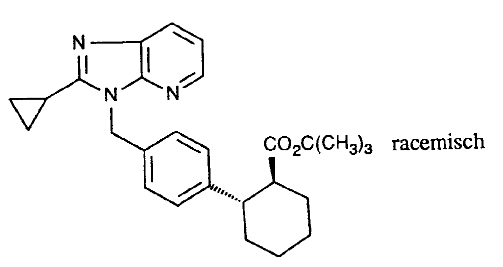

0,90 g (5,7 mmol) der Verbindung aus Beispiel V werden in 15 ml Dimethylformamid mit 0,17 g (5,7 mol) Natriumhydrid (80%ig, stabilisiert mit Paraffin) bei 0°C umgesetzt. Nach beendeter Wasserstoffentwicklung rührt man 20 Minuten nach und tropft dann bei 0°C 2,00 g (5,7 mmol) der Verbindung aus Beispiel IV in 20 ml Dimethylformamid zu. Unter Erwärmung auf 20°C läßt man 20 h nachrühren und extrahiert dann nach Zugabe von Wasser mehrfach mit Ether. Die organische Phase wird mit Natriumsulfat getrocknet, eingedampft und der Rückstand wird chromatographisch an Kieselgel 60 (Merck, Petrolether : Essigester = 1:1) gereinigt; Ausbeute: 0,4g (0,9 mmol).
R_{f} = 0,60 (Petrolether : Essigester = 1:2)

Analog zu Beispiel 1 werden die in Tabelle 1 aufgeführten Verbindungen hergestellt:

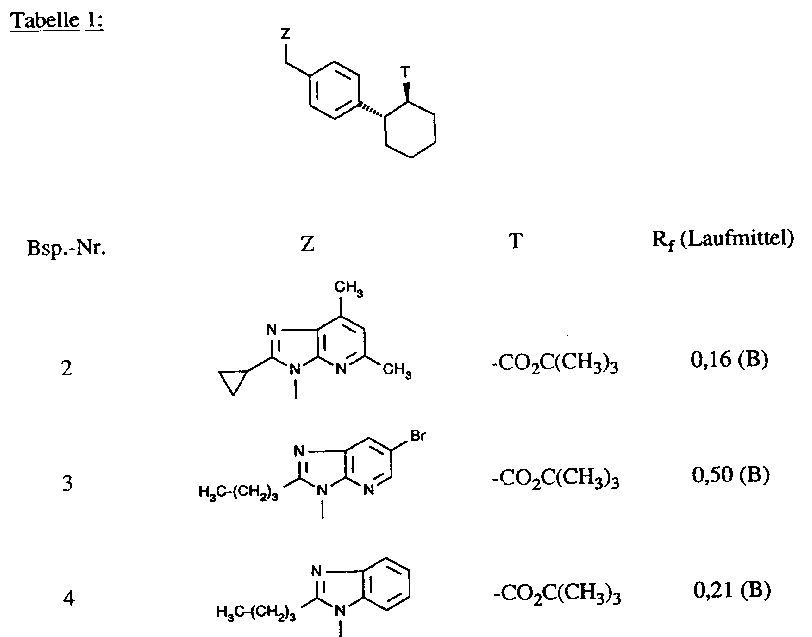

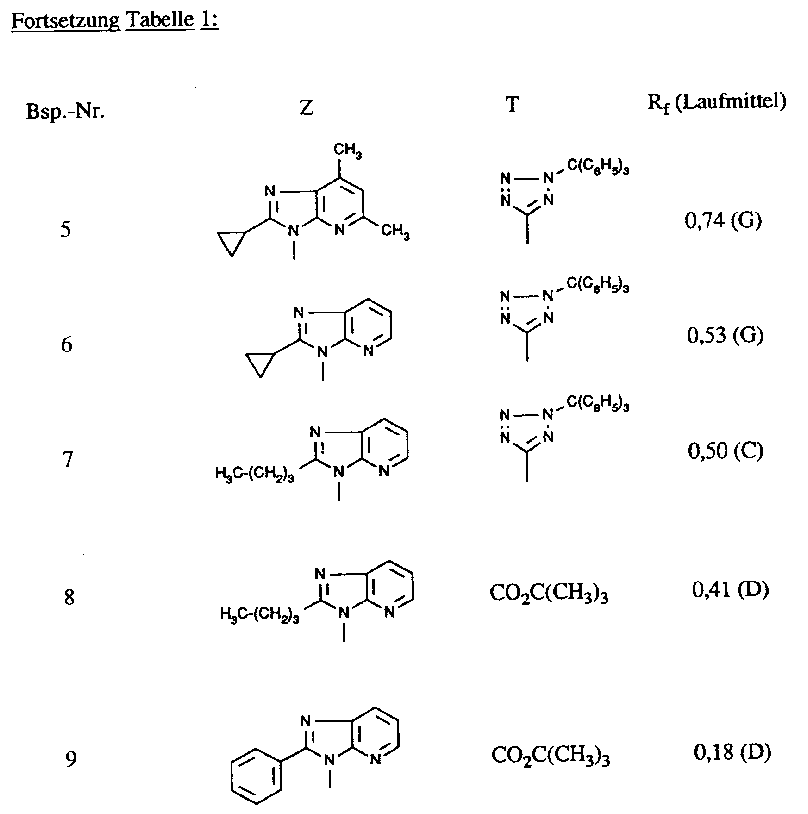

### Beispiel 10

### trans-2-{4-[(6-Cyclopropyl-imidazo[2,3-b]pyridin-7-yl-methyl]phenyl}-cyclohexan-1-carbonsäure

[0095]

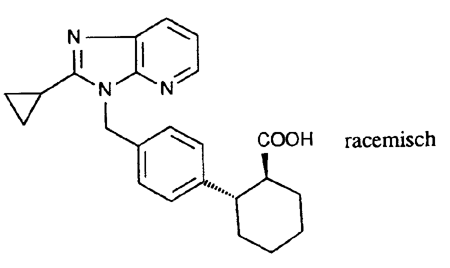

0,39 g (0,9 mmol) der Verbindung aus Beispiel 1 werden bei 20°C in 10 ml Dioxan mit 10 ml konzentrierter Salzsäure zur Reaktion gebracht. Nach 18 h stellt man mit 2 M Natronlauge auf pH = 13 und schüttelt einmal mit Ether. Nach Trennung der Phasen wird die wäßrige Lösung im Vakuum von orgnischen Lösemittelresten befreit und bei 0°C mit konzentrierter Salzsäure auf pH = 5 gestellt. Der dabei ausgefallene Niederschlag wird abgesaugt, mit Wasser gewaschen und im Hochvakuum über Natriumhydroxid und Phosphorpentoxid getrocknet.
Ausbeute: 0,28g (0.7 mmol)
R_{f} = 0,08 (Dihclormethan : Methanol = 10:19

In Analogie zu Beispiel 10 werden die in Tabelle 2 aufgeführten Verbindungen hergestellt:

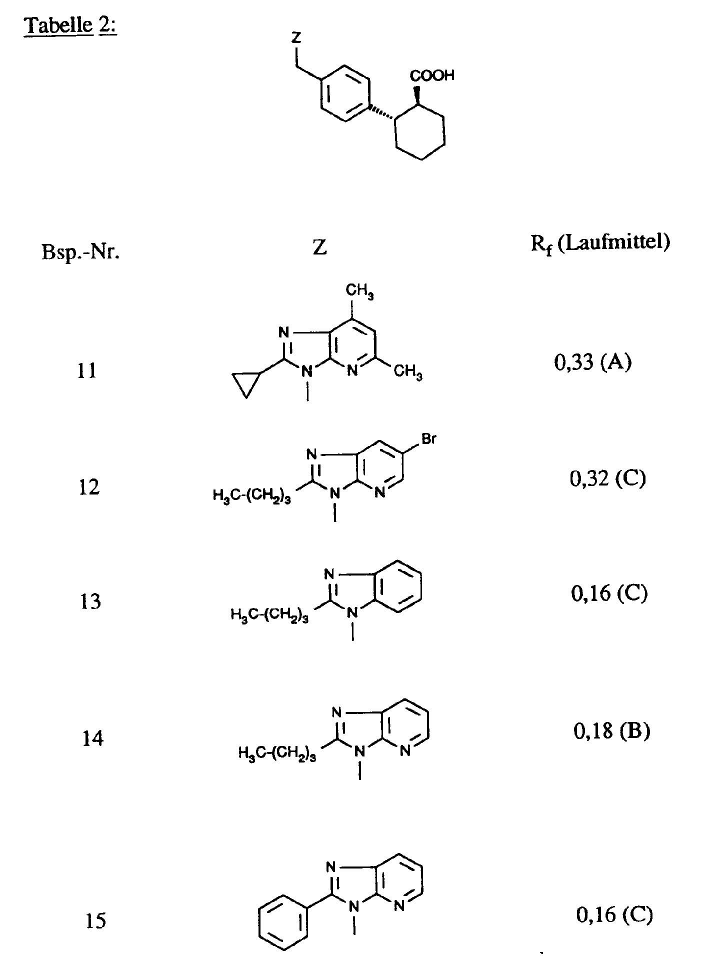

### Beispiele 16 und 17

### trans-2-{4-[(6-Cyclopropyl-imidazo[2,3-b]pyridin)-7-yl-methyl}-cyclohexan-1-carbonsäureN[(S)-phenylglycinol]amid

[0098]

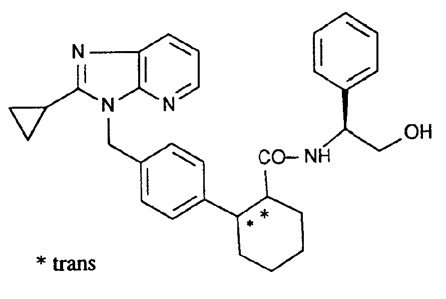

0,12 g (0,32 mmol) der Verbindung aus Beispiel 10 werden in 4 ml Tetrahydrofuran bei -30°C mit 89,6 µl (6,5 mmol) Triethylamin und 26,6 µl (0,35 mmol) Methansulfonsäurechlorid zur Reaktion gebracht. Nach 30 Minuten werden 52,6 mg (0,38 mmol) (S)-Phenylglycinol und 39 mg (0,32 mmol) 4-(N,N-Dimethylamino)-pyridin in 3 ml Tetrahydrofuran zugegeben und unter Erwärmung auf 20°C 19 h nachgerührt. Darauf gießt man auf wäßrige Natriumhydrogencarbonatlösung und extrahiert mehrfach mit Ether. Die etherishcen Extrakte werden mit Natriumsulfat getrocknet, eingedampft und der Rückstand an Kieselgel 60 (Merck, Dichlormethan : Methanol = 50:1) chromatographisch gereinigt.
Ausbeute:
   60 mg (0,12 mmol)/R_{f}= 0,63 (A) Diastereomer A (Beispiel 16)
   40 mg (0,08 mmol)/R_{f}= 0,59 (A) Diastereomer B (Beipsiel 17)

In Analogie zu den Vorschriften der Beispiele 16 und 17 werden die in Tabelle 3 aufgeführten Verbindungen hergestellt:

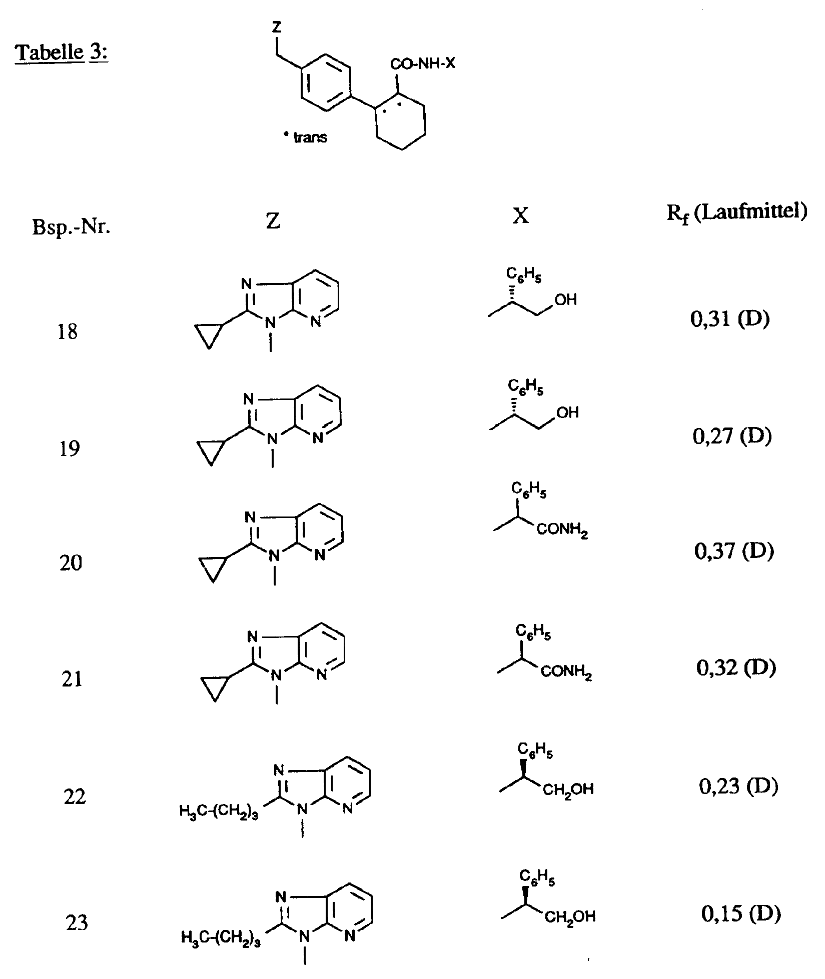

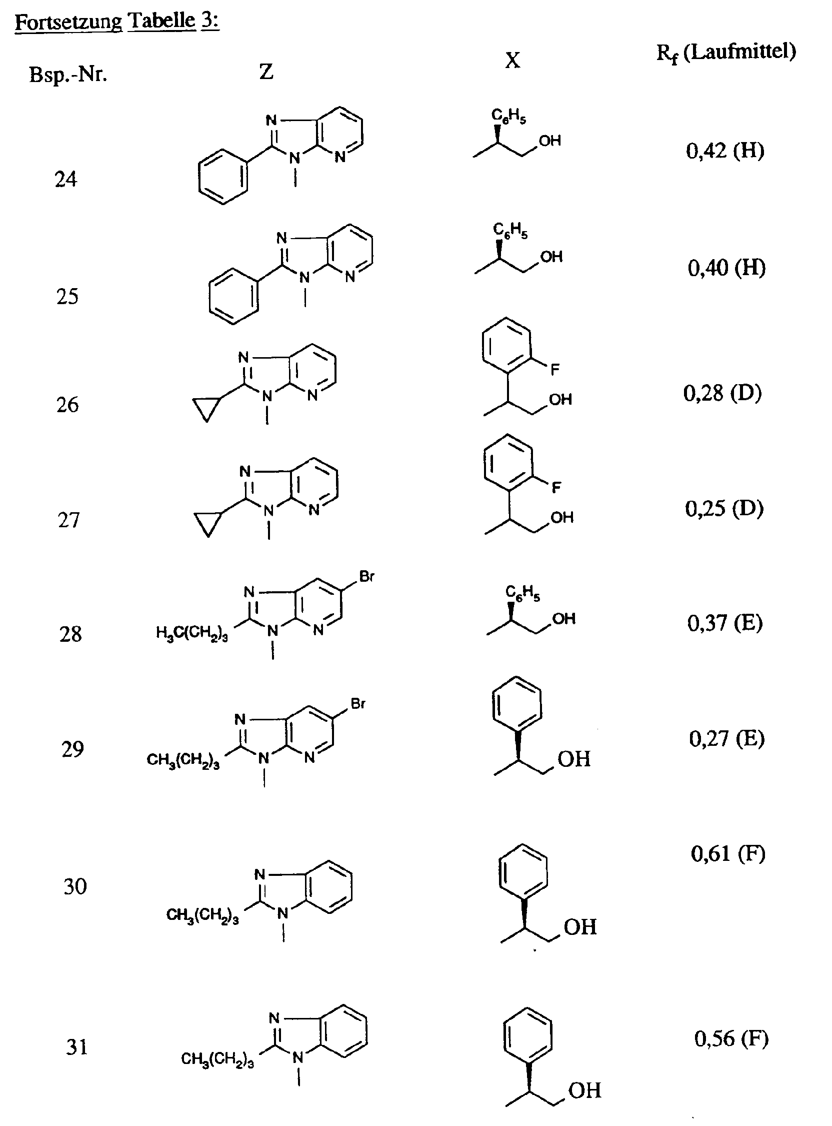

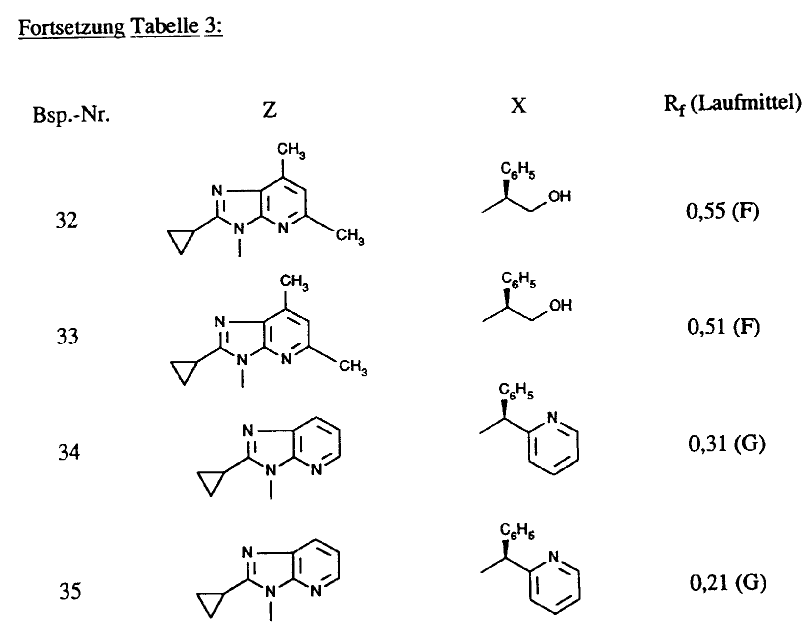

### Beispiel 36

### trans-2-{4-[(6-Cyclopropyl-imidazo[2,3-b]pyridin)-7-yl-methyl]phenyl}-cyclohexan-1-carbonsäure-N-(4-tolylsulfo- nyl)amid

[0101 ]

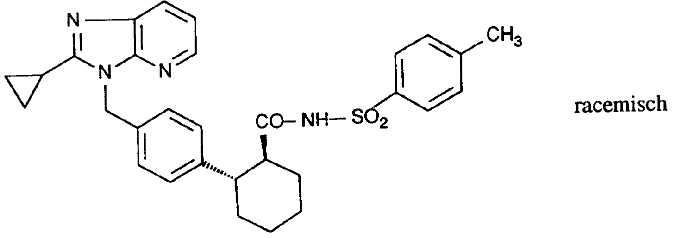

120 mg (0,32 mmol) der Verbindung aus Beispiel 10 werden in 4 ml Tetrahydrofuran mit 26,6 µl (0,35 mmol) Methansulfonäsurechlorid und 194 µl (1,4 mmol) Triethylamin umgesetzt (-20°C). Nach 2 h werden 66,0 mg (0,39 mmol) 4-Toluolsulfonsäureamid und 155 mg (1,28 mmol) 4-(N,N-Diethylamino)pyridin zum Reaktionsgemisch gegeben und 20 h nachgerührt. Man gießt auf wäßrige Natriumhydrogencarbonatlösung und extrahiert mit Ehter. die organische Phase wird mit Natriumsulfat getrocknet und eingedampft. Die chromatographische Reinigung des Rückstands (Kieselgel 60, Merck, Dichlormethan : Methanol = 50:1) liefert 34 mg (0,06 mmol) Produkt.
R_{f} = 0,45 (Petrolether : Essigester = 1:8).

### Beispiel 37

### 5-[trans-2-(2-Cyclopropyl-imidazo[2,3-b]pyridin-1-yl-methylphenyl)-cyclohex-1-yl]tetrazol

[0103]

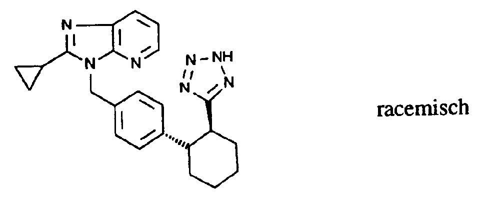

0,64 g (1,0 mmol) der Verbindung aus Beispiel IX werden in 7 ml Tetrahydrofuran mit 3 ml Wasser und 3 ml Trifluoressigsäure bei 20°C umgesetzt. Nach 2 Stunden verdünnt man mit Ether und extrahiert mit wäßriger Natronlauge (pH = 13). Die alkalische wäßrige Phase wird mit 1 M Salzsäure auf pH = 4,5 gestellt und der dabei anfallende Niederschlag abgesaugt, mit Wasser gewaschen und im Hochvakuum über Natriumhydroxid und Phosphorpentoxid getrocknet.
Ausbeute: 0,27 g (0,7 mmol).
R_{f}= 0,35 (Dichlormethan : Methanol = 10:1 )

In Analogie zur Vorschrift des Beispiels 37 können die in Tabelle 4 aufgeführten Verbindungen hergestellt werden:

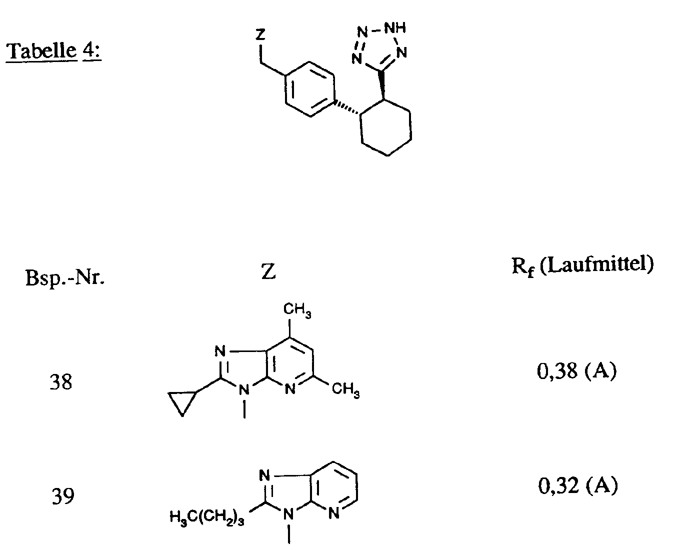

## Patentansprüche

1. Heterocyclisch substituierte Phenyl-cyclohexan-carbonsäurederivate der allgemeinen Formel (I) 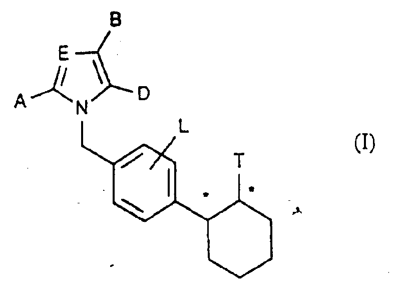 in welcher
A für Wasserstoff oder für Aryl mit 6 bis 10 Kohlenstoffatomen steht, das gegebenenfalls durch Hydroxy, Halogen, Trifluormethyl oder durch geradkettiges oder verzweigtes Alkyl oder Alkoxy mit jeweils bis zu 6 Kohlenstoffatomen substiutiert ist, oder
für geradkectiges oder verzweigtes Alkyl oder Alkenyl mit jeweils bis zu 8 Kohlenstoffatomen steht, oder
für Cycloalkyl mit 3 bis 8 Kohlenstoffatomen steht,
E für ein Stickstoffatom oder für einen Rest der Formel -CR¹ steht,
worin
R¹ die oben angegebene Bedeutung von A hat und mit dieser gleich oder verschieden ist,
B und D gemeinsam einen Rest der Formel 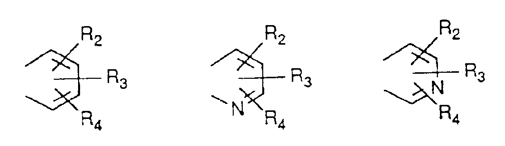 bilden, 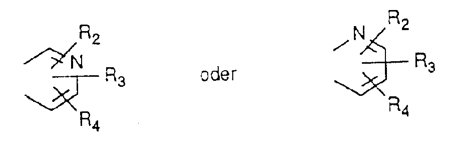 worin
R², R³ und R⁴ gleich oder verschieden sind und die oben angegebene Bedeutung von A haben und mit dieser gleich oder verschieden sind, oder Halogen bedeuten,
L für Wasserstoff, Halogen, Nitro, Hydroxy, Trifluormethyl, Trifluormethoxy, geradkettiges oder verzweigtes Alkyl, Alkoxy oder Alkoxycarbonyl mit jeweils bis zu 6 Kohlenstoffatomen, Cyano oder Carboxy steht,
T für einen Rest der Formel 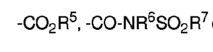 oder 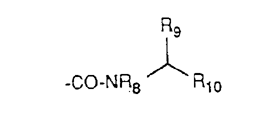 steht,
worin
R⁵ Wasserstoff, geradkettiges oder verzweigtes Alkyl mit bis zu 8 Kohlenstoffatomen, Cycloalkyl mit 3 bis 7 Kohlenstoffatomen oder Phenyl bedeutet,
R⁶ und R⁸ gleich oder verschieden sind und Wasserstoff oder geradkettiges oder verzweigtes Alkyl mit bis zu 6 Kohlenstoffatomen bedeuten,
R⁷ Trifluormethyl oder geradkettiges oder verzweigtes Alkyl mit bis zu 6 Kohlenstoffatomen, Benzyl oder Phenyl bedeutet, die gegebenenfalls durch geradkettiges Alkyl mit bis zu 6 Kohlenstoffatomen substituiert sind,
R⁹ Aryl mit 6 bis 10 Kohlenstoffatomen bedeutet, das gegebenenfalls bis zu 2-fach gleich oder verschieden durch Halogen, Hydroxy, geradkettiges oder verzweigtes Alkyl, Alkoxy oder Alkoxycarbonyl mit jeweils bis zu 6 Kohlenstoffatomen, Carboxy, Phenoxy oder C₃-C₆-Cycloalkyloxy substituiert ist,
R¹⁰ eine Gruppe der Formel -CH₂-OR¹¹, -C0₂R¹², -CO-NR¹³R¹⁴ oder Pyridyl bedeutet, worin
R¹¹ Wasserstoff oder geradkettiges oder verzweigtes Alkyl mit bis zu 8 Kohlenstoffatomen bedeutet,
R¹² Wasserstoff, geradkettiges oder verzweigtes Alkyl mit bis zu 8 Kohlenstoffatomen, Phenyl oder Cycloalkyl mit 3 bis 6 Kohlenstoffatomen bedeutet,
R¹³ und R¹⁴ gleich oder verschieden sind und Wasserstoff, geradkettiges oder verzweigtes Alkyl mit bis zu 8 Kohlenstoffatomen oder Phenyl bedeuten,
oder
T für Tetrazolyl steht, das gegebenenfalls durch Triphenylmethyl oder geradkettiges oder verzweigtes Alkyl mit bis zu 4 Kohlenstoffatomen substituiert ist,
gegebenenfalls in einer stereoisomeren Form, und deren physiologisch unbedenkliche Salze.

2. Heterocyclisch substituierte Phenyl-cyclohexan-carbonsäurederivate nach Anspruch 1
A für Wasserstoff oder für Phenyl steht, das gegebenenfalls durch Hydroxy, Fluor, Chlor, Brom, Trifluormethyl oder durch geradkettiges oder verzweigtes Alkyl oder Alkoxy mit jeweils bis zu 4 Kohlenstoffatomen substituiert ist, oder
für geradkettiges oder verzweigtes Alkyl oder Alkenyl mit jeweils bis zu 6 Kohlenstoffatomen steht, oder
für Cyclopropyl, Cyclobutyl, Cyclopentyl, Cyclohexyl oder Cycloheptyl steht,
E für ein Stickstoffatom oder für einen Rest der Formel -CR¹ steht,
worin
R¹ die oben angegebene Bedeutung von A hat und mit dieser gleich oder verschieden ist,
B und D gemeinsam einen Rest der Formel 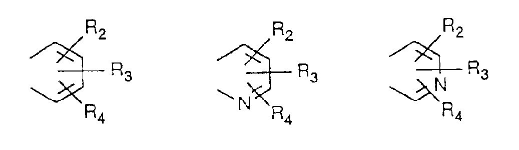 bilden, 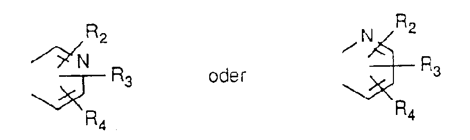 worin
R², R³ und R⁴ gleich oder verschieden sind und die oben angegebene Bedeutung von A haben und mit dieser gleich oder verschieden sind, oder Fluor, Chlor oder Brom bedeuten,
L für Wasserstoff, Fluor, Chlor, Brom, Trifluormethyl, Carboxy oder geradkettiges oder verzweigtes Alkyl, Alkoxy oder Alkoxycarbonyl mit jeweils bis zu 4 Kohlenstoffatomen steht,
T für einen Rest der Formel 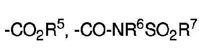 oder 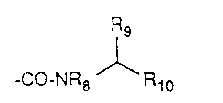 steht,
worin
R⁵ Wasserstoff, geradkettiges oder verzweigtes Alkyl mit bis zu 6 Kohlenstoffatomen, Cyclopropyl, Cyclopentyl, Cyclohexyl oder Phenyl bedeutet,
R⁶ und R⁸ gleich oder verschieden sind und Wasserstoff oder geradkettiges oder verzweigtes Alkyl mit bis zu 4 Kohlenstoffatomen bedeuten,
R⁷ Trifluormethyl oder geradkettiges oder verzweigtes Alkyl mit bis zu 4 Kohlenstoffatomen, Benzyl oder Phenyl bedeutet, die gegebenenfalls durch geradkettiges oder verzweigtes Alkyl mit bis zu 4 Kohlenstoffatomen substituiert sind,
R⁹ Phenyl bedeutet, das gegebenenfalls durch Fluor, Chlor, Brom, Hydroxy, geradkettiges oder verzweigtes Alkyl, Alkoxy oder Allzoxycarbonyl mit jeweils bis zu 4 Kohlenstoffatomen, Carboxy oder Phenoxy substituiert ist,
R¹⁰ eine Gruppe der Formel -CH₂-OR¹¹, -C0₂R¹², -CO-NR¹³R¹⁴ oder Pyridyl bedeutet, worin
R¹¹ Wasserstoff oder geradkettiges oder verzweigtes Alkyl mit bis zu 6 Kohlenstoffatomen bedeutet,
R¹² Wasserstoff, geradkettiges oder verzweigtes Alkyl mit bis zu 6 Kohlenstoffatomen, Phenyl, Cyclopropyl, Cyclopentyl oder Cyclohexyl bedeutet,
R¹³ und R¹⁴ gleich oder verschieden sind und Wasserstoff, geradkettiges oder verzweigtes Alkyl mit bis zu 6 Kohlenstoffatomen, Cyclopropyl, Cylcopentyl, Cylcohexyl oder Phenyl bedeuten, oder
T für Tetrazolyl steht, das gegebenenfalls durch Triphenylmethyl oder Methyl substituiert ist,
gegebenenfalls in einer stereoisomeren Form, und deren physiologisch unbedenkliche Salze.

3. Heterocyclisch substituierte Phenyl-cyclohexan-carbonsäurederivate nach Anspruch 1
A für Wasserstoff oder
für geradkettiges oder verzweigtes Alkyl mit bis zu 4 Kohlenstoffatomen steht, oder
für Phenyl oder Cyclopropyl, Cyclopentyl oder Cyclohexyl steht,
E für ein Stickstoffatom steht,
B und D gemeinsam einen Rest der Formel 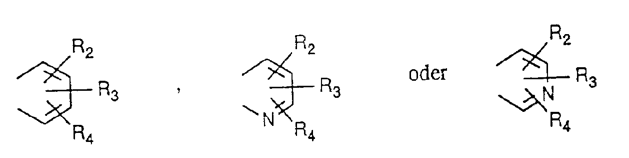 bilden,
worin
R², R³ und R⁴ gleich oder verschieden sind und die oben angegebene Bedeutung von A haben und mit dieser gleich oder verschieden sind, oder Fluor, Chlor oder Brom bedeuten,
L für Wasserstoff, Fluor, Chlor, Trifluormethyl oder Methyl steht,
T für einen Rest der Formel 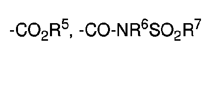 oder 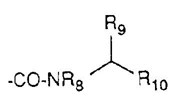 steht,
worin
R⁵ Wasserstoff, geradkettiges oder verzweigtes Alkyl mit bis zu 4 Kohlenstoffatomen bedeutet,
R⁶ und R⁸ gleich oder verschieden sind und Wasserstoff oder Methyl bedeuten,
R⁷ Trifluormethyl oder Methyl, Ethyl, Benzyl, p-Tolyl oder Phenyl bedeutet,
R⁹ Phenyl bedeutet, das gegebenenfalls durch Fluor, Chlor, Brom, Hydroxy, geradkettiges oder verzweigtes Alkyl, Alkoxy oder Alkoxycarbonyl mit jeweils bis zu 3 Kohlenstoffatomen, Carboxy oder Phenoxy substituiert ist,
R¹⁰ eine Gruppe der Formel -CH₂-OR¹¹, -CO₂-R¹², -CO-NR¹³R¹⁴ oder Pyridyl bedeutet,
worin
R¹¹ Wasserstoff oder geradkettiges oder verzweigtes Alkyl mit bis zu 4 Kohlenstoffatomen bedeutet,
R¹² Wasserstoff, geradkettiges oder verzweigtes Alkyl mit bis zu 4 Kohlenstoffatomen bedeutet,
R¹³ und R¹⁴ gleich oder verschieden sind und Wasserstoff, geradkettiges oder verzweigtes Alkyl mit bis zu 4 Kohlenstoffatomen bedeuten.
oder
T für Tetrazolyl steht, das gegebenenfalls durch Triphenylmethyl oder Methyl substituiert ist,
gegebenenfalls in einer stereoisomeren Form, und deren physiologisch unbedenkliche Salze.

4. Heterocyclisch substituierte Phenyl-cyclohexan-carbonsäurederivate nach Anspruch 1
A für Wasserstoff, geradkettiges oder verzweigtes Alkyl mit bis zu 4 Kohlenstoffatomen oder für Phenyl oder Cyclopropyl steht,
E für ein Stickstoffatom steht,
B und D gemeinsam einen Rest der Formel 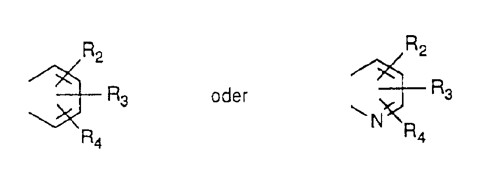 bilden,
worin
R², R³ und R⁴ gleich oder verschieden sind und Methyl, Wasserstoff, Fluor, Chlor oder Brom bedeuten,
L für Wasserstoff, Fluor oder Chlor steht,
T für einen Rest der Formel 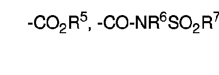 oder 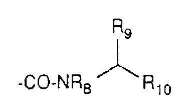 steht,
worin
R⁵ Wasserstoff oder geradkettiges oder verzweigtes Alkyl mit bis zu 4 Kohlenstoffatomen bedeutet,
R⁶ und R⁸ gleich oder verschieden sind und Wasserstoff oder Methyl bedeuten,
R⁷ Trifluormethyl, Methyl oder p-Tolyl bedeutet,
R⁹ Phenyl bedeutet, das durch Fluor, Chlor substituiert sein kann, und
R¹⁰ eine Gruppe der Formel -CH₂0H, oder -CONH₂ oder Pyridyl bedeutet, oder
T für Tetrazolyl steht, das gegebenenfalls durch Triphenylmethyl substituiert ist,
gegebenenfalls in einer stereoisomeren Form, und deren physiologisch unbedenkliche Salze.

5. Heterocyclisch substituierte Phenyl-cyclohexan-carbonsäurederivate nach den Ansprüchen 1 bis 4 zur Anwendung bei der Behandlung von Krankheiten.

6. Verfahren zur Herstellung von heterocyclisch substituierten Phenyl-cyclohexan-carbonsäurederivate nach den Ansprüchen 1 bis 4, dadurch gekennzeichnet, daß man
Verbindungen der allgemeinen Formel (II) 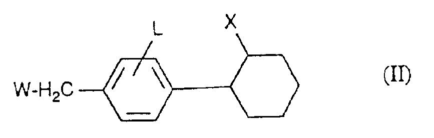 in welcher
L die oben angegebene Bedeutung hat,
W für eine typische Abgangsgruppe wie beispielsweise Chlor, Brom, Jod, Tosylat oder Mesylat, vorzugsweise für Brom steht und
X für Cᵢ-C₆-Alkoxycarbonyl oder für die Triphenylmethyl-tetrazolyl-1-yl-Gruppe steht,
zunächst mit Verbindungen der allgemeinen Formel (III) 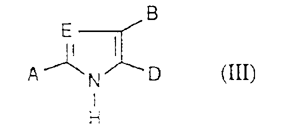 in welcher
A, B, D und E die oben anregebene Bedeutung haben,
in inerten Lösemitteln, gegebenenfalls in Anwesenheit einer Base und gegebenenfalls unter Schutzgasatmosphäre zu Verbindungen der allgemeinen Formel (IV) 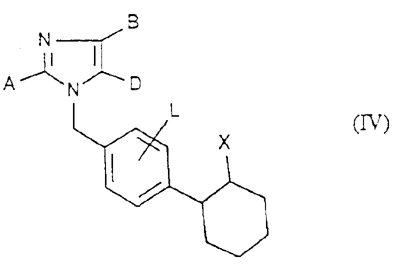 in welcher
A, B, D, L und X die oben angegebene Bedeutung haben,
umsetzt, und gegebenenfalls ausgehend von den entsprechenden Carbonsäuren, nach vorgeschalteter Hydrolyse und/oder Aktivierung, anschließend mit Sulfonaminen oder Aminen der allgemeinen Formeln (V) und (Va) 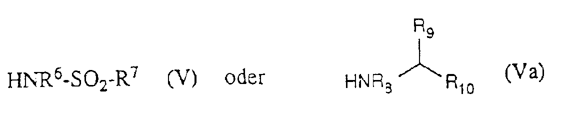 in welchen
_{R}⁶, _{R}⁷, _{R}⁸, R⁹ und R¹⁰ die oben angegebene Bedeutung haben,
gegebenenfalls in Anwesenheit einer Base und/oder eines Dehydratisierungs
mittels in inerten Lösemitteln amidiert,
und im Fall der freien Tetrazole, gegebenenfalls die Triphenylmethylgruppe nach üblichen Methoden mit Säuren, vorzugsweise mit Trifluoressigsäure oder Salzsäure in Dioxan, abspaltet,
gegebenenfalls die Stereoisomeren trennt, und im Fall der Herstellung der physiologisch unbedenklichen Salze mit einer entsprechenden Base oder Säure umsetzt.

7. Arzneimittel enthaltend mindestens ein heterocyclisch substituiertes Phenyl-cyclohexan-carbonsäurederivat nach den Ansprüchen 1 bis 4.

8. Arzneimittel nach Anspruch 7 zur Behandlung von arterieller Hypertonie und Atherosklerose.

9. Verwendung von heterocyclisch substituierten Phenyl-cyclohexan-carbonsäurederivate nach den Ansprüchen 1 bis 4 zur Herstellung von Arzneimitteln.

10. Verfahren zur Herstellung von Arzneimitteln nach Anspruch 7 oder 11, dadurch gekennzeichnet, daß man die heterocyclisch substituierten Phenyl-cyclohexan-carbonsäurederivate gegebenenfalls mit Hilfe von geeigneten Hilfs-und Trägerstoffen in eine geeignete Applikationsform überführt.

11. Arzneimittel nach Anspruch 7 zur Behandlung von ischämischen Gehirnerkrankungen und Ödemen.

## Claims

1. Heterocyclically substituted phenyl-cyclohexane-carboxylic acid derivatives of the general formula (I) 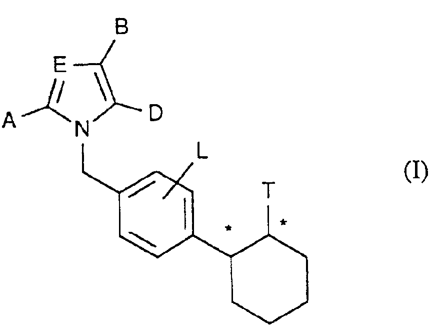 in which
A represents hydrogen or aryl having 6 to 10 carbon atoms, which is optionally substituted by hydroxyl, halogen or trifluoromethyl or by straight-chain or branched alkyl or alkoxy in each case having up to 6 carbon atoms, or
represents straight-chain or branched alkyl or alkenyl in each case having up to 8 carbon atoms, or
represents cycloalkyl having 3 to 8 carbon atoms,
E represents a nitrogen atom or a radical of the formula -CR¹,
in which
R¹ has the abovementioned meaning of A and is identical to or different from this,
B and D together form a radical of the formula 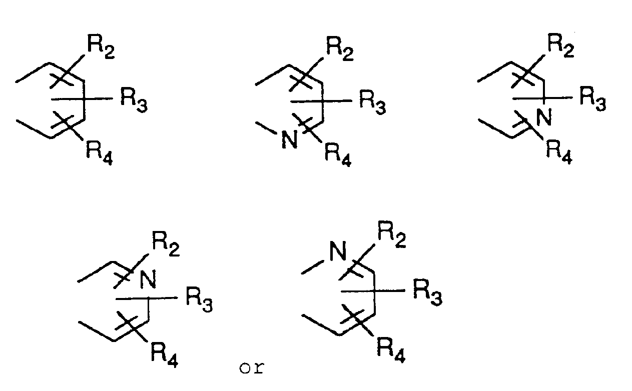 in which
R², R³ and R⁴ are identical or different and have the abovementioned meaning of A and are identical to or different from this, or denote halogen,
L represents hydrogen, halogen, nitro, hydroxyl, trifluoromethyl, trifluoromethoxy, straight-chain or branched alkyl, alkoxy or alkoxycarbonyl in each case having up to 6 carbon atoms, cyano or carboxyl,
T represents a radical of the formula 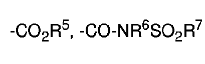 or 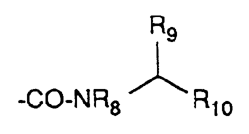 in which
R⁵ denotes hydrogen, straight-chain or branched alkyl having up to 8 carbon atoms, cycloalkyl having 3 to 7 carbon atoms, or phenyl,
R⁶ and R⁸ are identical or different and denote hydrogen or straight-chain or branched alkyl having up to 6 carbon atoms,
R⁷ denotes trifluoromethyl or straight-chain or branched alkyl having up to 6 carbon atoms, or benzyl or phenyl, each of which is optionally substituted by straight-chain alkyl having up to 6 carbon atoms, R⁹ denotes aryl having 6 to 10 carbon atoms, which is optionally substituted up to 2 times by identical or different substituents from the group consisting of halogen, hydroxyl, straight-chain or branched alkyl, alkoxy or alkoxycarbonyl in each case having up to 6 carbon atoms, carboxyl, phenoxy and C₃-C₆-cycloalkoxy,
R¹⁰ denotes a group of the formula -CH²-OR¹¹, -C0₂R¹², -CO-NR¹³R¹⁴ or pyridyl, in which
R¹¹ denotes hydrogen or straight-chain or branched alkyl having up to 8 carbon atoms,
R¹² denotes hydrogen, straight-chain or branched alkyl having up to 8 carbon atoms, or phenyl or cycloalkyl having 3 to 6 carbon atoms,
R¹³ and R¹⁴ are identical or different and denote hydrogen, straight-chain or branched alkyl having up to 8 carbon atoms, or phenyl, or
T represents tetrazolyl, which is optionally substituted by triphenylmethyl or straight-chain or branched alkyl having up to 4 carbon atoms,
if appropriate in a stereoisomeric form, and their physiologically acceptable salts.

2. Beterocyclically substituted phenyl-cyclohexane-carboxylic acid derivatives according to Claim 1 in which
A represents hydrogen or phenyl which is optionally substituted by hydroxyl, fluorine, chlorine, bromine or trifluoromethyl or by straight-chain or branched alkyl or alkoxy in each case having up to 4 carbon atoms, or
represents straight-chain or branched alkyl or alkenyl in each case having up to 6 carbon atoms, or
represents cyclopropyl, cyclobutyl, cyclopentyl, cyclohexyl or cycloheptyl,
E represents a nitrogen atom or a radical of the formula -CR¹, in which
R¹ has the abovementioned meaning of A and is identical to or different from this,
B and D together form a radical of the formula 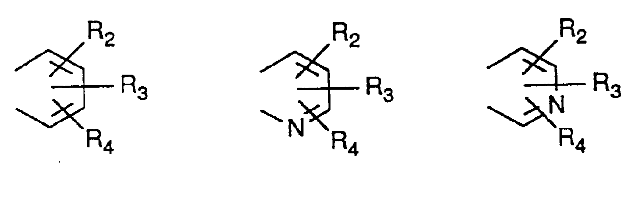 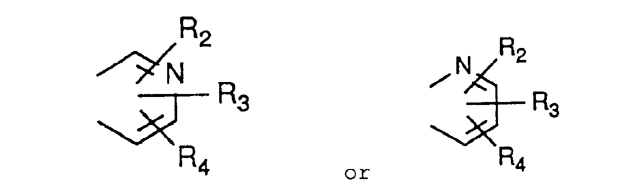 in which
R², R³ and R⁴ are identical or different and have the abovementioned meaning of A and are identical to or different from this, or denote fluorine, chlorine or bromine,
L represents hydrogen, fluorine, chlorine, bromine, trifluoromethyl, carboxyl or straight-chain or branched alkyl, alkoxy or alkoxycarbonyl in each case having up to 4 carbon atoms,
T represents a radical of the formulae 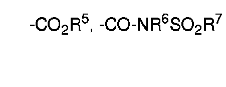 or 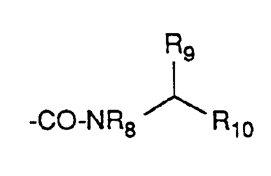 in which
R⁵ denotes hydrogen, straight-chain or branched alkyl having up to 6 carbon atoms, cyclopropyl, cyclopentyl, cyclohexyl or phenyl,
R⁶ and R⁸ are identical or different and denote hydrogen or straight-chain or branched alkyl having up to 4 carbon atoms,
R⁷ denotes trifluoromethyl or straight-chain or branched alkyl having up to 4 carbon atoms, or benzyl or phenyl, each of which is optionally substituted by straight-chain or branched alkyl having up to 4 carbon atoms, R⁹ denotes phenyl which is optionally substituted by fluorine, chlorine, bromine, hydroxyl, straight-chain or branched alkyl, alkoxy or alkoxycarbonyl in each case having up to 4 carbon atoms, carboxyl or phenoxy,
R¹⁰ denotes a group of the formula -CH₂-OR¹¹, -C0₂R¹², -CO-NR¹³R¹⁴ or pyridyl, in which
R¹¹ denotes hydrogen or straight-chain or branched alkyl having up to 6 carbon atoms, R¹² denotes hydrogen, straight-chain or branched alkyl having up to 6 carbon atoms, phenyl, cyclopropyl, cyclopentyl or cyclohexyl,
R¹³ and R¹⁴ are identical or different and denote hydrogen, straight-chain or branched alkyl having up to 6 carbon atoms, cyclopropyl, cyclopentyl, cyclohexyl or phenyl, or
T represents tetrazolyl, which is optionally substituted by triphenylmethyl or methyl,
if appropriate in a stereoisomeric form, and their physiologically acceptable salts.

3. Heterocyclically substituted phenyl-cyclohexane-carboxylic acid derivatives according to Claim 1 in which
A represents hydrogen or
represents straight-chain or branched alkyl having up to 4 carbon atoms, or
represents phenyl or cyclopropyl, cyclopentyl or cyclohexyl,
E represents a nitrogen atom,
B and D together form a radical of the formula 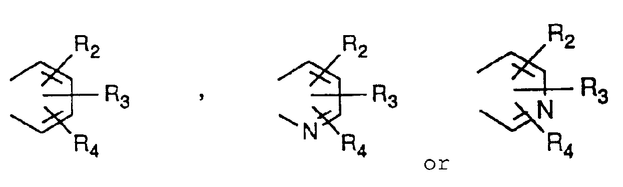 in which
R², R³ and R⁴ are identical or different and have the abovementioned meaning of A and are identical to or different from this, or denote fluorine, chlorine or bromine,
L represents hydrogen, fluorine, chlorine, trifluoromethyl or methyl,
T represents a radical of the formula 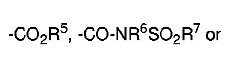 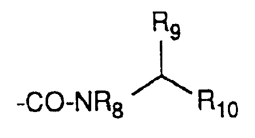 in which
R⁵ denotes hydrogen or straight-chain or branched alkyl having up to 4 carbon atoms,
R⁶ and R^{B} are identical or different and denote hydrogen or methyl,
R⁷ denotes trifluoromethyl or methyl, ethyl, benzyl, p-tolyl or phenyl,
R⁹ denotes phenyl which is optionally substituted by fluorine, chlorine, bromine, hydroxyl, straight-chain or branched alkyl, alkoxy or alkoxycarbonyl in each case having up to 3 carbon atoms, carboxyl or phenoxy,
R¹⁰ denotes a group of the formula -CH₂-OR¹¹, -CO₂R¹², -CO-NR¹³R¹⁴ or pyridyl, in which
R¹¹ denotes hydrogen or straight-chain or branched alkyl having up to 4 carbon atoms,
R¹² denotes hydrogen or straight-chain or branched alkyl having up to 4 carbon atoms,
R¹³ and R¹⁴ are identical or different and denote hydrogen or straight-chain or branched alkyl having up to 4 carbon atoms, or
T represents tetrazolyl, which is optionally substituted by triphenylmethyl or methyl,
if appropriate in a stereoisomeric form, and their physiologically acceptable salts.

4. Heterocyclically substituted phenyl-cyclohexane-carboxylic acid derivatives according to Claim 1 in which
A represents hydrogen, straight-chain or branched alkyl having up to 4 carbon atoms, or represents phenyl or cyclopropyl,
E represents a nitrogen atom,
B and D together form a radical of the formula 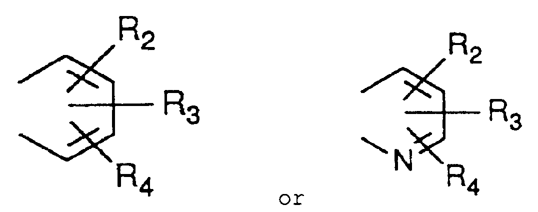 in which
R², R³ and R⁴ are identical or different and denote methyl, hydrogen, fluorine, chlorine or bromine,
L represents hydrogen, fluorine or chlorine,
T represents a radical of the formula 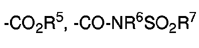 or 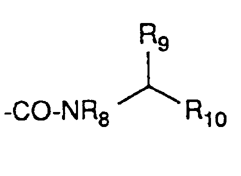 in which
R⁵ denotes hydrogen or straight-chain or branched alkyl having up to 4 carbon atoms,
R⁶ and R are identical or different and denote hydrogen or methyl,
R⁷ denotes trifluoromethyl, methyl or p-tolyl,
R⁹ denotes phenyl which can be substituted by fluorine or chlorine, and
R¹⁰ denotes a group of the formula -CH₂0H or -CONH₂ or pyridyl, or
T represents tetrazolyl, which is optionally substituted by triphenylmethyl,
if appropriate in a stereoisomeric form, and their physiologically acceptable salts.

5. Heterocyclically substituted phenyl-cyclohexane-carboxylic acid derivatives according to Claims 1 to 4 for use in the treatment of diseases.

6. Process for the preparation of heterocyclically substituted phenyl-cyclohexane-carboxylic acid derivatives according to Claims 1 to 4, characterized in that
compounds of the general formula (II) 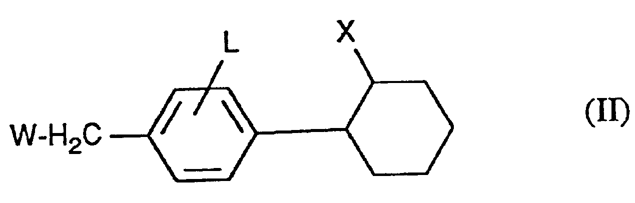 in which
L has the abovementioned meaning,
W represents a typical leaving group such as, for example, chlorine, bromine, iodine, tosylate or mesylate, preferably bromine and
X represents C₁-C₆-alkoxycarbonyl or the triphenylmethyl-tetrazol-1-yl group,
are reacted first with compounds of the general formula (III) 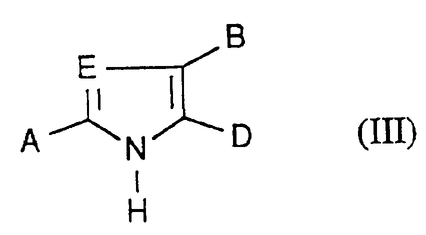 in which
A, B, D and E have the abovementioned meaning,
in inert solvents, if appropriate in the presence of a base and if appropriate under a protective gas atmosphere, to give compounds of the general formula (IV) 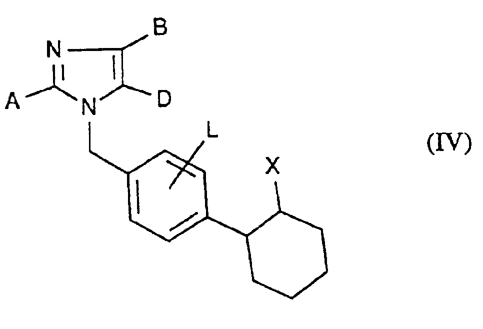 in which
A, B, D, L and X have the abovementioned meaning,
and if appropriate starting from the corresponding carboxylic acids, the products are subsequently amidated in inert solvents, after prior hydrolysis and/or activation, with sulphonamides or amines of the general formulae (V) and (Va) 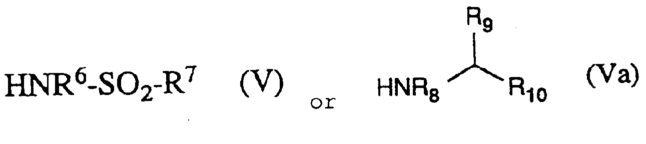 in which
_{R}⁶, _{R}⁷, _{R}⁸, _{R}⁹ and R¹⁰ have the abovementioned
meaning,
if appropriate in the presence of a base and/or of a dehydrating agent,
and in the case of the free tetrazoles, if appropriate the triphenylmethyl group is removed by customary methods using acids, preferably using trifluoroacetic acid or hydrochloric acid in dioxane,
if appropriate the stereoisomers are separated, and in the case of the preparation of the physiologically acceptable salts reacted with an appropriate base or acid.

7. Medicaments containing at least one heterocyclically substituted phenyl-cyclohexane-carboxylic acid derivative according to Claims 1 to 4.

8. Medicaments according to Claim 7 for the treatment of arterial hypertension and atherosclerosis.

9. Use of heterocyclically substituted phenyl-cyclohexane-carboxylic acid derivatives according to Claims 1 to 4 for the production of medicaments.

10. Process for the production of medicaments according to Claim 7 or 11, characterized in that the heterocyclically substituted phenyl-cyclohexane-carboxylic acid derivatives are converted into a suitable administration form, if appropriate with the aid of suitable auxiliaries and excipients.

11. Medicaments according to claim 7 for the treatment of ischaemic cerebral diseases.

## Revendications

1. Dérivés d'acides phényl-cyclohexane-carboxyliques à substituant hétérocyclique, de formule générale (I) 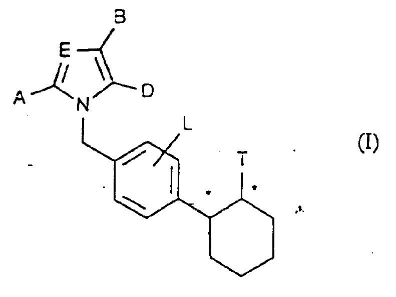 dans laquelle
A représente l'hydrogène ou un groupe aryle ayant 6 à 10 atomes de carbone, qui est éventuellement substitué par un radical hydroxy, halogéno, trufluorométhyle ou par un radical alkyle ou un radical alkoxy linéaire ou ramifié ayant chacun jusqu'à 6 atomes de carbone, ou bien
un groupe alkyle ou alcényle linéaire ou ramifié ayant chacun jusqu'à 8 atomes de carbone, ou bien
un groupe cycloalkyle ayant 3 à 8 atomes de carbone,
E représente un atome d'azote ou un reste de formule -CR¹,
dans laquelle
R¹ a la définition indiquée ci-dessus pour A et est identique à A ou en est différent,
B et D forment ensemble un reste de formule 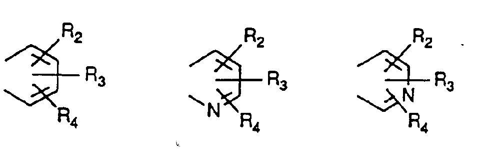 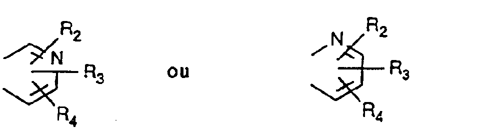 où
R², R³ et R⁴ sont identiques ou différents et ont la définition indiquée ci-dessus pour A et sont identiques à A ou en sont différents ou représentent un halogène,
L représente l'hydrogène, un halogène, un groupe nitro, hydroxy, trifluorométhyle, trifluorométhoxy, un groupe alkyle, alkoxy ou alkoxycarbonyle linéaire ou ramifié ayant chacun jusqu'à 6 atomes de carbone, un groupe cyano ou carboxy,
T représente un reste de formule 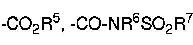 ou 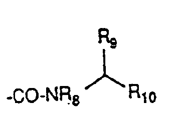 où
R⁵ est l'hydrogène, un groupe alkyle linéaire ou ramifié ayant jusqu'à 8 atomes de carbone, un groupe cycloalkyle ayant 3 à 7 atomes de carbone ou un groupe phényle,
R⁶ et R⁸ sont identiques ou différents et représentent l'hydrogène ou un groupe alkyle linéaire ou ramifié ayant jusqu'à 6 atomes de carbone,
R⁷ est un groupe trifluorométhyle ou un groupe alkyle linéaire ou ramifié ayant jusqu'à 6 atomes de carbone, un groupe benzyle ou phényle, qui sont substitués, le cas échéant, par un radical alkyle linéaire ayant jusqu'à 6 atomes de carbone,
R⁹ est un groupe aryle ayant 6 à 10 atomes de carbone, qui est substitué, le cas échéant, jusqu'à 2 fois identiques ou différentes par un radical halogéno, hydroxy, un radical alkyle, alkoxy ou alkoxycarbonyle linéaire ou ramifié ayant chacun jusqu'à 6 atomes de carbone, carboxy, phénoxy ou cycloalkyloxy en C₃ à C₆,
R¹⁰ est un groupe de formule -CH₂-OR¹¹, -CO₂R¹², -CO-NR¹³R¹⁴ ou pyridyle, où
R¹¹ représente l'hydrogène ou un groupe alkyle linéaire ou ramifié ayant jusqu'à 8 atomes de carbone,
R¹² est l'hydrogène, un groupe alkyle linéaire ou ramifié ayant jusqu'à 8 atomes de carbone, phényle ou cycloalkyle ayant 3 à 6 atomes de carbone,
R¹³ et R¹⁴ sont identiques ou différents et représentent l'hydrogène, un groupe alkyle linéaire ou ramifié ayant jusqu'à 8 atomes de carbone ou un groupe phényle, ou bien
T est un groupe tétrazolyle qui est substitué, le cas échéant, par un radical triphénylméthyle ou un radical alkyle linéaire ou ramifié ayant jusqu'à 4 atomes de carbone,
le cas échéant sous une forme stéréo-isomère, et leurs sels acceptables du point de vue physiologique.

2. Dérivés d'acides phényl-cyclohexane-carboxyliques à substituant hétérocyclique suivant la revendication 1, dans lesquels
A représente l'hydrogène ou un groupe phényle qui est substitué, le cas échéant, par un radical hydroxy, fluoro, chloro, bromo, trifluorométhyle ou par un radical alkyle ou un radical alkoxy linéaire ou ramifié ayant chacun jusqu'à 4 atomes de carbone, ou bien
un groupe alkyle ou un groupe alcényle linéaire ou ramifié ayant chacun jusqu'à 6 atomes de carbone, ou bien
un groupe cyclopropyle, cyclobutyle, cyclopentyle, cyclohexyle ou cycloheptyle,
E représente un atome d'azote ou un reste de formule -CR¹, dans laquelle
R¹ a la définition indiquée ci-dessus pour A et est identique à A ou en est différent,
B et D représentent ensemble un reste de formule 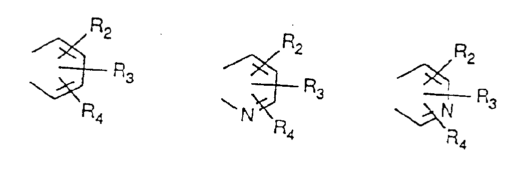 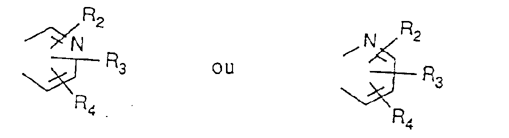 dans laquelle
R², R³ et R⁴ sont identiques ou différents et ont la définition indiquée ci-dessus pour A et sont identiques à A ou en sont différents, ou représentent le fluor, le chlore ou le brome,
L est l'hydrogène, le fluor, le chlore, le brome, un groupe trifluorométhyle, carboxy ou un groupe alkyle, un groupe alkoxy ou un groupe alkoxycarbonyle linéaire ou ramifié ayant chacun jusqu'à 4 atomes de carbone,
T est un reste de formule 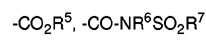 ou 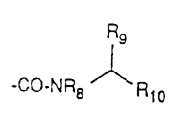 dans laquelle
R⁵ est l'hydrogène, un groupe alkyle linéaire ou ramifié ayant jusqu'à 6 atomes de carbone, un groupe cyclopropyle, cyclopentyle, cyclohexyle ou phényle,
R⁶ et R^{B} sont identiques ou différents et représentent l'hydrogène ou un groupe alkyle linéaire ou ramifié ayant jusqu'à 4 atomes de carbone,
R⁷ est un groupe trifluorométhyle ou un groupe alkyle linéaire ou ramifié ayant jusqu'à 4 atomes de carbone, un groupe benzyle ou un groupe phényle, qui sont substitués, le cas échéant, par un radical alkyle linéaire ou ramifié ayant jusqu'à 4 atomes de carbone,
R⁹ désigne un groupe phényle qui est substitué, le cas échéant, par du fluor, du chlore, du brome, un radical hydroxy, un radical alkyle, alkoxy ou alkoxycarbonyle linéaire ou ramifié ayant chacun jusqu'à 4 atomes de carbone, carboxy ou phénoxy,
R¹⁰ est un groupe de formule -CH₂-OR¹¹, -C0₂R¹², -CO-NR¹³R¹⁴ ou un groupe pyridyle, où
R¹¹ est l'hydrogène ou un groupe alkyle linéaire ou ramifié ayant jusqu'à 6 atomes de carbone,
R¹² est l'hydrogène, un groupe alkyle linéaire ou ramifié ayant jusqu'à 6 atomes de carbone, phényle, cyclopropyle, cyclopentyle ou cyclohexyle,
R¹³ et R¹⁴ sont identiques ou différents et représentent l'hydrogène, un groupe alkyle linéaire ou ramifié ayant jusqu'à 6 atomes de carbone, cyclopropyle, cyclopentyle, cyclohexyle ou phényle, ou bien
T est un groupe tétrazolyle qui est substitué, le cas échéant, par un radical triphénylméthyle ou méthyle,
éventuellement sous une forme stéréo-isomère, et leurs sels acceptables du point de vue physiologique.

3. Dérivés d'acides phényl-cyclohexane-carboxyliques à substituant hétérocyclique suivant la revendication 1 dans lesquels
A est l'hydrogène ou
un groupe alkyle linéaire ou ramifié ayant jusqu'à 4 atomes de carbone, ou
un groupe phényle ou un groupe cyclopropyle, cyclopentyle ou cyclohexyle,
E représente un atome d'azote,
B et D forment ensemble un reste de formule 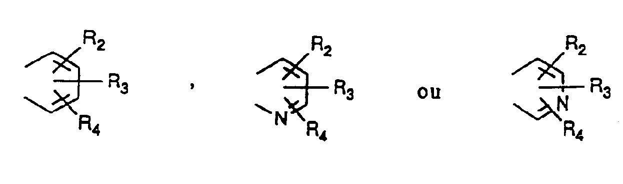 dans laquelle
R², R³ et R⁴ sont identiques ou différents et ont la définition indiquée ci-dessus pour A et sont identiques à A ou en sont différents, ou représentent le fluor, le chlore ou le brome,
L est l'hydrogène, le fluor, le chlore, un groupe trifluorométhyle ou méthyle,
T est un reste de formule 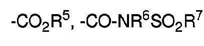 ou 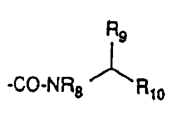 dans laquelle
R⁵ est l'hydrogène, un groupe alkyle linéaire ou ramifié ayant jusqu'à 4 atomes de carbone,
R⁶ et R⁸ sont identiques ou différents et représentent l'hydrogène ou un groupe méthyle,
R⁷ est un groupe trifluorométhyle ou un groupe méthyle, éthyle, benzyle, p-tolyle ou phényle,
R⁹ est un groupe phényle qui est substitué, le cas échéant par un radical fluoro, chloro, bromo, hydroxy, un radical alkyle, alkoxy ou alkoxycarbonyle linéaire ou ramifié ayant chacun jusqu'à 3 atomes de carbone, carboxy ou phénoxy,
R¹⁰ est un groupe de formule -CH₂-OR¹¹, -CO₂R¹², -CO-NR¹³R¹⁴ ou pyᵣi_{d}yₗₑ, où
R¹¹ est l'hydrogène ou un groupe alkyle linéaire ou ramifié ayant jusqu'à 4 atomes de carbone,
R¹² est l'hydrogène, un groupe alkyle linéaire ou ramifié ayant jusqu'à 4 atomes de carbone,
R¹³ et R¹⁴ sont identiques ou différents et représentent l'hydrogène, un groupe alkyle linéaire ou ramifié ayant jusqu'à 4 atomes de carbone,
ou bien
T est un groupe tétrazolyle qui est substitué, le cas échéant par un radical triphénylméthyle ou méthyle,
éventuellement sous une forme stéréo-isomère, et leurs sels acceptables du point de vue physiologique.

4. Dérivés d'acides phényl-cyclohexane-carboxyliques à substituant hétérocyclique suivant la revendication 1, dans lesquels
A est l'hydrogène, un groupe alkyle linéaire ou ramifié ayant jusqu'à 4 atomes de carbone, ou un groupe phényle ou cyclopropyle,
E est un atome d'azote,
B et D forment ensemble un reste de formule 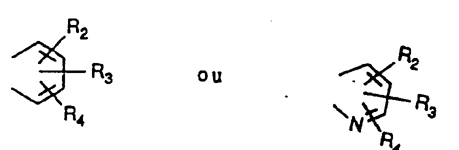 dans laquelle
R², R³ et R⁴ sont identiques ou différents et représentent un groupe méthyle, l'hydrogène, le fluor, le chlore ou le brome,
L est l'hydrogène, le fluor ou le chlore,
T est un reste de formule 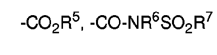 ou 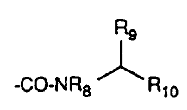 dans laquelle
R⁵ est l'hydrogène ou un groupe alkyle linéaire ou ramifié ayant jusqu'à 4 atomes de carbone,
R⁶ et R^{B} sont identiques ou différents et représentent l'hydrogène ou un groupe méthyle,
R⁷ est un groupe trifluorométhyle, méthyle ou p-tolyle,
R⁹ est un groupe phényle qui peut être substitué par du fluor ou du chlore, et
R¹⁰ est un groupe de formule -CH₂0H ou -CONH₂ ou un groupe pyridyle, ou bien
T est un groupe tétrazolyle qui est substitué, le cas échéant par un radical triphénylméthyle,
éventuellement sous une forme stéréo-isomère, et leurs sels acceptables du point de vue physiologique.

5. Dérivés d'acides phényl-cyclohexane-carboxyliques à substituant hétérocyclique suivant les revendications 1 à 4, destinés à être utilisés dans le traitement de maladies.

6. Procédé de production de dérivés d'acides phénylcyclohexane-carboxyliques à substituant hétérocyclique suivant les revendications 1 à 4, caractérisé en ce qu'on commence par faire réagir
des composés de formule générale (II) 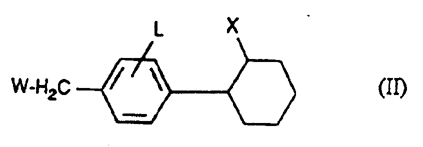 dans laquelle
L a la définition indiquée ci-dessus,
W est un groupe partant typique tel que, par exemple, chlore, brome, iode, tosylate ou mésylate, de préférence brome et
X est un groupe (alkoxy en Ci à C₆)carbonyle ou un groupe triphénylméthyl-tétrazolyl-1-yle,
avec des composés de formule générale (III) 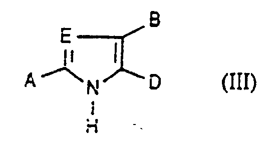 dans laquelle
A, B, D et E ont la définition indiquée ci-dessus,
dans des solvants inertes, éventuellement en présence d'une base et, le cas échéant, sous atmosphère de gaz protecteur, pour former des composés de formule générale (IV) 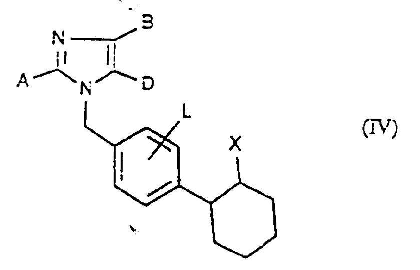 dans laquelle
A, B, D, L et X ont la définition indiquée ci-dessus,
dont on effectue ensuite, le cas échéant, l'amidation en partant des acides carboxyliques correspondants, après hydrolyse et/ou activation préalables, avec des sulfonamines ou des amines de formules générales (V) et (Va) 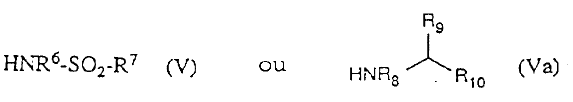 dans lesquelles
_{R}⁶, _{R}⁷, _{R}⁸, R⁹ et R¹⁰ ont la définition indiquée ci-dessus,
éventuellement en présence d'une base et/ou d'un agent déshydratant, dans des solvants inertes,
et dans le cas des tétrazoles libres, on élimine éventuellement le groupe triphénylméthyle par des modes opératoires classiques avec des acides, de préférence avec l'acide trifluoracétique ou l'acide chlorhydrique dans le dioxanne,
on sépare éventuellement les stéréo-isomères et, dans le cas de la préparation des sels acceptables du point de vue physiologique, on les fait réagir avec une base ou un acide correspondant.

7. Médicament contenant au moins un dérivé d'acide phényl-cyclohexane-carboxylique à substituant hétérocyclique suivant les revendications 1 à 4.

8. Médicament suivant la revendication 7, destiné au traitement de l'hypertonie artérielle et de l'athérosclérose.

9. Utilisation de dérivés d'acides phényl-cyclohexane-carboxyliques à substituant hétérocyclique suivant les revendications 1 à 4 pour la préparation de médicaments.

10. Procédé de production de médicaments suivant la revendication 7 ou 11, caractérisé en ce qu'on fait prendre une forme d'administration appropriée à des dérivés d'acides phényl-cyclohexane-carboxyliques à substituant hétérocyclique à l'aide de substances auxiliaires et de supports appropriés.

11. Médicament suivant la revendication 7, destiné au traitement de troubles cérébraux ischémiques et d'oedèmes.
